# EUROPEAN PATENT APPLICATION

(11) **EP 3 881 843 A1**
(43) Date of publication of application: **22.09.2021**
(21) Application number: 19885251.9
(22) Date of filing: 15.11.2019
(51) Int. Cl.: A61K 31/4545, A61K 31/47, A61K 31/4985, A61K 45/00, A61P 43/00, A61P 17/04, A61K 47/64, A61K 38/16

(54) **PROPHYLACTIC OR THERAPEUTIC AGENT AND MEDICINAL COMPOSITION FOR IL-31-MEDIATED DISEASE**

(30) Priority: 15.11.2018 JP 2018215017; 06.08.2019 JP 2019144913
(71) Applicant: Kyushu University, National University Corporation, Nishi-ku Fukuoka-shi Fukuoka 819-0395 (JP); National University Corporation University Of Toyama, Toyama-shi, Toyama 930-8555 (JP)
(72) Inventor: FUKUI Yoshinori, Fukuoka-shi, Fukuoka 819-0395 (JP); SAKATA Daiji, Fukuoka-shi, Fukuoka 819-0395 (JP); URUNO Takehito, Fukuoka-shi, Fukuoka 819-0395 (JP); ANDOH Tsugunobu, Toyama-shi, Toyama 930-0194 (JP)
(74) Representative: EP&C
(86) International application number: PCT/JP2019/044886
(87) International publication number: WO 2020/101017

(57) **Abstract**

A prophylactic or therapeutic agent for an IL-31 mediated disease, said agent comprising a neurokinin B signal blocker.

## Description

### Technical Field

The present invention relates to a prophylactic or therapeutic agent and a medicinal composition for an TL-31-mediated disease.

Priority is claimed on Japanese Patent Application No. 2018-215017, filed on November 15, 2018 and Japanese Patent Application No. 2019-144913, filed on August 6, 2019, the contents of which are incorporated herein by references.

### Background Art

Interleukin-31 (IL-31) is a cytokine expressed in various human tissues, and it has been reported that interleukin-31 is associated with a chronically pruritic skin disorder such as atopic dermatitis (for example, refer to Non Patent Documents 1 and 2).

Atopic dermatitis is a chronic disease with itchy eczema that worsens and relieves repeatedly. Many patients have an enhanced response to allergens. In recent years, the number of patients with atopic dermatitis has been increasing, and since atopic dermatitis significantly impairs the quality of life of patients, there is an urgent need to develop a therapeutic method for atopic dermatitis.

Furthermore, intradermal administration of IL-31 in mice induces scratching behavior. The scratching behavior in mice can be used as a model of scratching behavior induced by itching in atopic dermatitis.

The severity of atopic dermatitis is known to correlate with serum IL-31 levels. It was also reported that administration of an anti-IL-31 receptor antibody alleviates pruritus in patients with atopic dermatitis. From these findings, it is considered that the causative substance that induces itching in atopic dermatitis is IL-31.

It has also been reported that IL-31 mediates the pathophysiology of various diseases by inducing the expression of other inflammatory cytokines in addition to the induction of pruritus. IL-31 -mediated diseases such as atopic dermatitis, pruritus in atopic dermatitis, dermatomyositis, pruritus in dermatomyositis, chronic dermatitis, allergic contact dermatitis, dermatitis herpetiformis, pruritus in cutaneous T cell lymphoma, prurigo nodularis, prurigo chronica multiformis, urticaria pigmentosa, and bullous pemphigoid have been reported (for example, refer to Non Patent Documents 1 to 8). Dermatomyositis is an autoimmune inflammatory myopathy with a rash, in which the severity of a skin symptom correlates with itching, and it has been reported that the expression of IL-31 and IL-31 receptors is enhanced in lesioned skin with itching (Non Patent Document 3). Cutaneous T cell lymphoma (CTCL) is a group of malignant lymphomas that occur in the skin, and is a disease in which cells from which tumors are derived are T cells. Regarding CTCL, it has been reported that disease progression and severity of pruritus correlate with IL-31 and IL-31 receptor expression levels in lesioned skin and serum IL-31 levels (Non Patent Document 4). Regarding prurigo nodularis and allergic contact dermatitis, it has been reported that the enhanced expression of IL-31 is observed in skin tissues (Non Patent Documents 5 and 6), and regarding urticaria pigmentosa, it has been reported that serum IL-31 levels correlate with the severity of disease and pruritus (Non Patent Document 7). Furthermore, it has been reported that IL-31 levels in serum and tissues were increased in patients with dermatitis herpetiformis or bullous pemphigoid, and many IL-31-positive cells in the skin tissues at lesion sites of these patients were observed (Non Patent Document 8).

The inventors have previously clarified that the production of IL-31 from CD4+ T cells was enhanced in mice deficient for the Dedicator of cytokinesis 8 (Dock8) gene and transgenic for a rearranged T cell receptor (TCR) gene (for example, refer to Non Patent Document 9).

Here, AND can be used as the rearranged TCR. The AND is the TCR that recognizes a peptide (SEQ ID NO: 1) consisting of 88th to 103rd amino acids of moth cytochrome c (MCC), which forms a complex with an MHC class II I-Eκ molecule. On the other hand, AND-expressed T cells normally differentiate and mature in the thymus of mice that express the I-Ab molecule, but AND TCR shows relatively high autoreactivity to the I-Ab molecule.

Regarding the genetic background of C57BL/6 mice expressing the I-Ab molecule, Dock8 knockout and AND transgenic (Dock8-/- AND Tg) mice show enhanced scratching behaviors and symptoms of dermatitis. Thus, Dock 8-/- AND Tg mice can be used as a non-human animal model of IL-31-mediated disease such as atopic dermatitis, such as for atopic dermatitis.

### Citation List

### Non Patent Documents

[Non Patent Document 1]
   Furue M, et al. Emerging role of interleukin-31 and interleukin-31 receptor in pruritus in atopic dermatitis. Allergy 73:29-36, 2018.
[Non Patent Document 2]
   Bagci IS, Ruzicka T. IL-31: A new key player in dermatology and beyond. J Allergy Clin Immunol. 141:858-866, 2018.
[Non Patent Document 3]
   Kim HJ, et al. Itch in dermatomyositis: the role of increased skin interleukin-31. Br J Dermatol. 179:669-678, 2018.
[Non Patent Document 4]
   Nattkemper LA. et al. Cutaneous T-cell Lymphoma and Pruritus: The Expression of IL-31 and its Receptors in the Skin. Acta Derm Venereol. 96:894-898, 2016.
[Non Patent Document 5]
   Sonkoly E, et al. IL-31: a new link between T cells and pruritus in atopic skin inflammation. J Allergy Clin Immunol. 117:411-417, 2006.
[Non Patent Document 6]
   Neis MM, et al. Enhanced expression levels of IL-31 correlate with IL-4 and IL-13 in atopic and allergic contact dermatitis. J Allergy Clin Immunol. 118:930-937, 2006.
[Non Patent Document 7]
   Lange M, et al. Interleukin-31 polymorphisms and serum IL-31 level in patients with mastocytosis: correlation with clinical presentation and pruritus. Acta Derm Venereol. 97:47-53, 2017.
[Non Patent Document 8]
   Bonciani D, et al. Serum levels and tissue expression of interleukin-31 in dermatitis herpetiformis and bullous pemphigoid. J Dermatol Sci. 87:210-212, 2017.
[Non Patent Document 9]
   Yamamura K et al., The transcription factor EPAS1 links DOCK 8 deficiency to atopic skin inflammation via IL-31 induction. Nature Communications 8:13946, 2017.

### Summary of Invention

### Technical Problem

As described above, IL-31 is considered to be a typical factor that induces itching in inflammatory diseases such as atopic dermatitis. However, the mechanism by which IL-31 induces itching has not been fully elucidated. Therefore, an object of the present invention is to clarify a molecular mechanism by which IL-31 induces itching, and to provide a prophylactic or therapeutic technique for an IL-31-mediated disease such as atopic dermatitis.

### Solution to Problem

The present invention includes the following aspects.
[1] A prophylactic or therapeutic agent for an IL-31-mediated disease comprising a neurokinin B signal blocker.
[2] The prophylactic or therapeutic agent according to [1], in which the IL-31-mediated disease includes atopic dermatitis, pruritus in atopic dermatitis, dermatomyositis, pruritus in dermatomyositis, chronic dermatitis, allergic contact dermatitis, dermatitis herpetiformis, pruritus in cutaneous T cell lymphoma, prurigo nodularis, prurigo chronica multiformis, urticaria pigmentosa, or bullous pemphigoid.
[3] The prophylactic or therapeutic agent according to [1], in which the IL-31-mediated disease is atopic dermatitis.
[4] The prophylactic or therapeutic agent according to any one of [1] to [3], in which the neurokinin B signal blocker is a neurokinin 3 receptor antagonist.
[5] The prophylactic or therapeutic agent according to [4], in which the antagonist is a compound represented by General Formula (1), (2), or (3) or a salt thereof, or a solvate thereof, 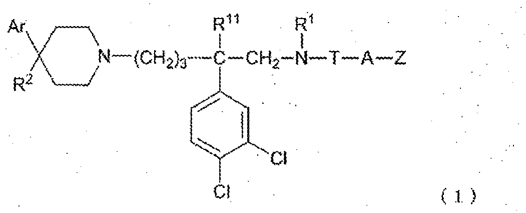 [in Formula (1),
   Ar represents a piperidinyl group, a pyridin-2-yl group, a phenyl group, or a phenyl group substituted with a halogen atom, a methyl group, or an alkoxy group having 1 to 4 carbon atoms;
   R¹ represents a methyl group and R¹¹ represents a hydrogen atom, or R¹ and R¹¹ together represent a -(CH₂)₃- group or a -(CH₂)₂O- group;
   R² represents a hydroxyl group, a C₁₋₇ alkoxy group, a C₁₋₇ acyloxy group, a cyano group, a -NR⁶R⁴ group, a -NR³COR⁴ group, a -NR³COOR⁸ group, a -NR³SO₂R⁹ group, a -NR³CONR¹⁰R¹² group, a C₁₋₇ acyl group, a C₁₋₇ alkoxycarbonyl group, a - CONR¹⁰R¹² group, a CH₂OH group, a C₁₋₇ alkoxymethyl group, a C₁₋₇ acyloxymethyl group, a C₁₋₇ alkylaminocarbonyloxymethyl group, a -CH₂NR¹³R¹⁴ group, a - CH₂NR³COR⁴ group, a -CH₂NR³COOR^{R} group, a -CH₂NR³SO₂R⁹ group, or a - CH₂NR³CONR¹⁰R¹² group, R² constitutes a double bond between the carbon atom to which it is attached and the adjacent carbon atom of the piperidine ring, or Ar and R² together with a piperidine ring to which Ar and R² are bonded form a group represented by Formula (1a) or (1b), 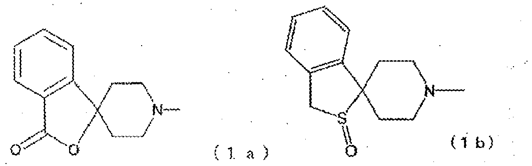
   where R³ represents a hydrogen atom or a C₁₋₄ alkyl group and R⁴ represents a hydrogen atom, a C₁₋₇ alkyl group, a phenyl group, a benzyl group, a pyridyl group, an unsubstituted C₃₋₇ cycloalkyl group, or a C₃₋₇ cycloalkyl group substituted with one or more methyl groups, or R³ and R⁴ together represent a -(CH₂)ₙ- group (where n is 3 or 4);
   T represents a methylene group, a carbonyl group, a -COO- group, or a - CONR⁵- group and A represents a single bond, a methylene group, an ethylene group, a propylene group, or a vinylene group, or -T-A- represents a -SO₂- group; and
   Z represents a phenyl group or a phenyl group substituted with one or more of a halogen atom, a C₁₋₄ alkyl group, a C₁₋₄ alkoxy group, and a nitro group,
   where R⁵ represents a hydrogen atom or a C₁₋₄ alkyl group,
   R⁶ represents a hydrogen atom or a C₁₋₇ alkyl group and R⁷ represents a hydrogen atom, a C₁₋₇ alkyl group, a C₃₋₇ cycloalkylmethyl group, a benzyl group, or a phenyl group, or R⁶ and R⁷ together with a nitrogen atom to which R⁶ and R⁷ are bonded form a heterocycle selected from the group consisting of an azetidine group, a pyrrolidine group, a piperidine group, a morpholine group, a thiomorpholine group, and a perhydroazepine group,
   R⁸ represents a C₁₋₇ alkyl group or a phenyl group,
   R⁹ represents a C₁₋₇ alkyl group, an amino group or an amino group substituted with one or two C₁₋₇ alkyl groups, a phenyl group or a phenyl group substituted with one or more groups selected from the group consisting of a halogen atom, a C₁₋₇ alkyl group, a trifluoromethyl group, a hydroxyl group, a C₁₋₇ alkoxy group, a carboxyl group, a C₁₋₇ alkoxycarbonyl group, a C₁₋₇ alkylcarbonyloxy group, a cyano group, a nitro group, an amino group, and an amino group substituted with one or two C₁₋₇ alkyl groups (where, in a case where a plurality of groups are selected, the plurality of groups are the same as or different from each other),
   R¹⁰ represents a hydrogen atom or a C₁₋₇ alkyl group and R¹² represents a hydrogen atom, a C₁₋₇ alkyl group, a C₃₋₇ cycloalkyl group, a C₃₋₇ cycloalkylmethyl group, a hydroxyl group, a C₁₋₄ alkoxy group, a benzyl group, or a phenyl group, or R¹⁰ and R¹² together with a nitrogen atom to which R¹⁰ and R¹² are bonded form a heterocycle selected from the group consisting of an azetidine group, a pyrrolidine group, a piperidine group, a morpholine group, a thiomorpholine group, and a perhydroazepine group,
   R¹³ represents a hydrogen atom or a C₁₋₇ alkyl group,
   R¹⁴ represents a hydrogen atom, a C₁₋₇ alkyl group, a C₃₋₇ cycloalkylmethyl group, or a benzyl group,
   n3 is 2 or 3, and
   in a case where Ar is a phenyl group, R² is a hydroxyl group, and -T-A-Z is a benzoyl group, R¹ is not a methyl group; in a case where Ar is a phenyl group, R² is a - NHCOCH₃ group, and -T-A-Z is a benzoyl group, R¹ and R¹¹ together do not form a - (CH2)₃- group; in a case where Ar is a phenyl group, R² is a hydroxyl group, and -T-A-Z is a 3-methoxybenzyl group, R¹ and R¹¹ together do not form a -(CH₂)₃- group; and in a case where Ar is a phenyl group, R² is a -NHCOCH₃ group, and -T-A-Z is a benzyloxycarbonyl group, R¹ is not a methyl group], 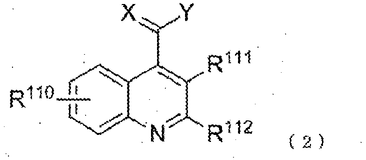
   [in Formula (2),
   Y is a group represented by Formula (2a) or (2b), 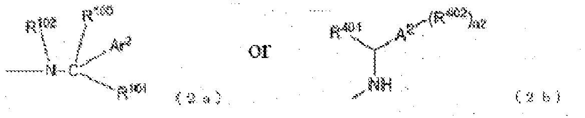
   where Ar² represents a phenyl group, a naphthyl group, or a C₅₋₇ cycloalkadienyl group, which is optionally substituted, or represents an optionally substituted single or condensed heterocyclic group having aromatic properties, having 5 to 12 ring atoms, and containing up to 4 heteroatoms selected from sulfur atoms, oxygen atoms, and nitrogen atoms in a ring or in each ring,
   R¹⁰⁰ represents a linear or branched C₁₋₈ alkyl group, a C₃₋₇ cycloalkyl group, a C₄₋₇ cycloalkylalkyl group, an optionally substituted phenyl group or a phenyl C₁₋₆ alkyl group, an optionally substituted 5-membered heteroaromatic ring containing up to 4 heteroatoms selected from oxygen atoms and nitrogen atoms, a hydroxy C₁₋₆ alkyl group, an amino C₁₋₆ alkyl group, a C₁₋₆ alkylaminoalkyl group, a di C₁₋₆ alkylaminoalkyl group, a C₁₋₆ acylaminoalkyl group, a C₁₋₆ alkoxyalkyl group, a C₁₋₆ alkylcarbonyl group, a carboxy group, a C₁₋₆ alkoxycarbonyl group, a C₁₋₆ alkoxycarbonyl C₁₋₆ alkyl group, an aminocarbonyl group, a C₁₋₆ alkylaminocarbonyl group, a di C₁₋₆ alkylaminocarbonyl group, or a halogeno C₁₋₆ alkyl group, or in a case of forming a ring with Ar², R¹⁰⁰ forms a group-(CH₂)ₚ- (where p is 2 or 3),
   R¹⁰¹ and R¹⁰² are the same as or different from each other, and each independently represents a hydrogen atom, a C₁₋₆ linear chain or branched alkyl group, or together forms a -(CH₂)ₙ₁- group (where n1 is 3, 4, or 5), or R¹⁰¹ and R¹⁰⁰ together form a group-(CH₂)_{q}- (where q is 2, 3, 4, or 5),
   R¹¹⁰ is R¹⁰³ or (R⁴⁰⁵)_{q1},
   R¹¹¹ is R¹⁰⁴ or a group represented by Formula (2c) or (2g), 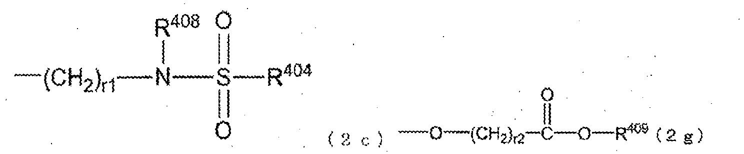
   R¹¹² is R¹⁰⁵ or a group represented by Formula (2d), 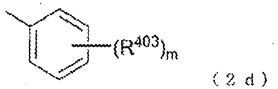
   R⁴⁰¹ is selected from a hydrogen atom, a C₁₋₄ alkyl group, a C₃₋₆ cycloalkyl group, and a C₁₋₄ alkylOC(O)-,
   A² is a phenyl group or a C₃₋₇ cycloalkyl group,
   each R⁴⁰² is independently selected from a hydrogen atom, -OH, -NH₂, -CN, a halogen atom, a C₁₋₆ alkyl group, a C₃₋₇ cycloalkyl group, a C₁₋₆ alkoxy group, and a C₁₋₆ alkoxy C₁₋₆ alkyl group,
   n2 is 1, 2, or 3,
   each R⁴⁰³ is independently selected from a hydrogen atom, -OH, -NH₂, -NO₂, - CN, a halogen atom, a C₁₋₆ alkyl group, a C₁₋₆ alkoxy group, and a C₁₋₆ alkoxy C₁₋₆ alkyl group,
   m is 1, 2, or 3,
   r1 is 0, 1, 2, or 3,
   r2 is 1, 2, or 3,
   R⁴⁰⁴ and R⁴⁰⁹ are selected from a C₁₋₄ alkyl group, a C₁₋₆ alkoxy C₁₋₆ alkyl group, a C₃₋₇ cycloalkyl group, and E-(CH₂)_{b}-, wherein E is -NR⁴⁰⁶R⁴⁰⁷, -SR⁴⁰⁶, a -SOC₁₋₆ alkyl group, a -SO₂C₁₋₆ alkyl group, N⁺(O⁻)R⁴⁰⁶R⁴⁰⁷, -NR⁴⁰⁶SO₂R⁴⁰⁷, an aryl group, and a N- or C-bonded 5- or 6-membered aromatic or non-aromatic heterocyclic ring having 1, 2, 3, or 4 nitrogen atoms or a N-oxide thereof, and b is 0, 1, 2, 3, 4, or 5,
   each R⁴⁰⁵ is independently selected from a hydrogen atom, -OH, -CN, a halogen, -R4⁰⁶, -OR⁴⁰⁶, -NR⁴⁰⁶R⁴⁰⁷, -SR⁴⁰⁶, -SOR⁴⁰⁶, and -SO₂R⁴⁰⁶,
   ql is 1, 2, or 3,
   R⁴⁰⁶ and R⁴⁰⁷ are each independently selected from a hydrogen atom, a C₁₋₆ linear or branched alkyl group, a C₂₋₆ linear or branched alkenyl group or alkynyl group, and a C₃₋₇ carbocyclic group having 0, 1, or 2 double or triple bonds, where the groups are unsubstituted or substituted with one or more groups selected from -OH, =O, -NH₂, - CN, a halogen atom, an aryl, and a C₁₋₃ alkoxy group,
   R⁴⁰⁸ is selected from a hydrogen atom, a C₁₋₅ linear or branched alkyl group, and a C₃₋₅ cycloalkyl group, where the groups are unsubstituted or substituted with one or more groups selected from -OH, =O, -NH₂, -CN, a halogen, an aryl group, and a C₁₋₃ alkoxy group,
   in a case where R⁴⁰⁴ is E-(CH₂)_{b}-, and E is a N- or C-bonded 5- or 6-membered aromatic or non-aromatic heterocyclic ring or a N-oxide thereof, E is unsubstituted or independently selected from -OH, =O, -NH₂, -CN, a halogen atom, a C₁₋₄ alkyl group, a C₁₋₄ alkoxy group, a C₁₋₄ alkyl-CO-, -NR⁴⁰⁶R⁴⁰⁷, an aryl, and a N- or C-bonded 5- or 6-membered aromatic or non-aromatic heterocyclic ring having 1, 2, 3, or 4 nitrogen atoms
   or a N-oxide thereof,
   in a case where R⁴⁰¹, R⁴⁰², R⁴⁰³, or R⁴⁰⁴ is an alkyl group, a cycloalkyl group, an alkoxy group, or an alkoxyalkyl group, the group is unsubstituted or has 1, 2, 3, 4, or 5 substituents each of which is independently selected from -OH, -NH₂, -CN, phenyl, and a halogen,
   R¹⁰³ and R¹⁰⁴ are the same as or different from each other, and are independently selected from a hydrogen atom, a C₁₋₆ linear or branched alkyl group, a C₁₋₆ alkenyl group, an aryl group, a C₁₋₆ alkoxy group, a hydroxy group, a halogen atom, a nitro group, a cyano group, a carboxy group, a carboxyamide group, a sulfonamide group, a C₁₋₆ alkoxycarbonyl group, a trifluoromethyl group, an acyloxy group, a phthalimide group, an amino group, a mono- or di-C₁₋₆ alkylamino group, an -O(CH₂)ᵣ-NW₂ group (where r is 2, 3, or 4, and W is a hydrogen atom or a C₁₋₆ alkyl group or forms a group 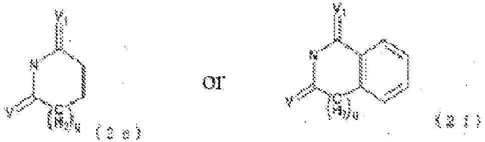
   with nitrogen adjacent thereto [in Formula (2e) or (2f), V and V₁ independently represent a hydrogen atom or an oxygen atom, and u is 0, 1, or 2]),
   an -O(CH₂)ₜ-OE₂ group (where t is 2, 3, or 4, and E is a hydrogen atom or a C₁₋₆ alkyl group), a hydroxyalkyl group, an aminoalkyl group, a mono- or di-alkylaminoalkyl group, an acylamino group, an alkylsulfonylamino group, an aminoacylamino group, and a mono- or di-alkylaminoacylamino group, up to 4 R¹⁰³ substituents are present in a quinoline nucleus, or R¹⁰⁴ forms a -(CH₂)ₑ- group (where e is 1, 2, or 3) in a case of forming a ring with R¹⁰⁵ as an aryl,
   R¹⁰⁵ represents a branched or linear C₁₋₆ alkyl group, a C₃₋₇ cycloalkyl group, a C₄₋₇ cycloalkylalkyl group, an optionally substituted aryl group, or an optionally substituted single or condensed heterocyclic group having aromatic properties, having 5 to 12 ring atoms, and containing up to 4 heteroatoms selected from sulfur atoms, oxygen atoms, and nitrogen atoms in a ring or in each ring, and
   X represents an oxygen atom, a sulfur atom, or =N-C=N], and 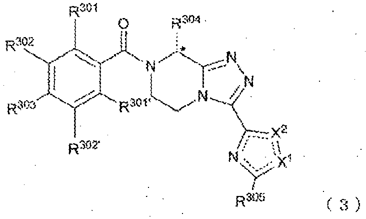
   [in Formula (3),
   R³⁰¹ is a hydrogen atom, a fluorine atom, or a methyl group,
   R^{301'} is a hydrogen atom,
   R³⁰² is a hydrogen atom, a fluorine atom, a chlorine atom, or a methoxy group,
   R^{302'} is a hydrogen atom or a fluorine atom,
   R³⁰³ is a hydrogen atom, a fluorine atom, a chlorine atom, a methyl group, a trifluoromethyl group, or a nitrile group,
   R³⁰⁴ is methyl, ethyl, n-propyl, hydroxyethyl, methoxyethyl, trifluoromethyl, difluoromethyl, or fluoromethyl,
   R³⁰⁵ is methyl, ethyl, methoxymethyl, trifluoromethyl, difluoromethyl, fluoromethyl, 1-fluoroethyl, 1,1-difluoroethyl, or 2,2,2-trifluoroethyl,
   X¹ is a nitrogen atom and X² is a sulfur atom or an oxygen atom, or X¹ is a sulfur atom and X² is a nitrogen atom, 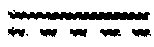
   represents a single bond or a double bond, depending on X¹ and X², and 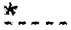
   represents an (R)-enantiomer or racemic compound of the compound of Formula (3)].
[6] The prophylactic or therapeutic agent according to [5], in which the compound represented by Formula (1) is osanetant.
[7] The prophylactic or therapeutic agent according to [5], in which the compound represented by Formula (1) is SSR-146977
[8] The prophylactic or therapeutic agent according to [5], wherein the compound represented by Formula (1) is SSR-241586.
[9] The prophylactic or therapeutic agent according to [5], in which the compound represented by Formula (1) is CS-003.
[10] The prophylactic or therapeutic agent according to [5], in which the compound represented by Formula (2) is talnetant.
[11] The prophylactic or therapeutic agent according to [5], in which the compound represented by Formula (2) is pavinetant.
[12] The prophylactic or therapeutic agent according to [5], in which the compound represented by Formula (2) is SB-235375.
[13] The prophylactic or therapeutic agent according to [5], in which the compound represented by Formula (3) is fezolinetant.
[14] The prophylactic or therapeutic agent according to any one of [1] to [3], in which the neurokinin B signal blocker is an inhibitor of a tachykinin processing enzyme or a decomposition accelerator of a tachykinin processing enzyme.
[15] The prophylactic or therapeutic agent according to [14], in which the inhibitor is an inhibitor of proprotein convertase subtilisin/kexin 1, proprotein convertase subtilisin/kexin 2, carboxypeptidase E, or peptidyl-glycine alpha-amidating monooxygenase.
[16] The prophylactic or therapeutic agent according to any one of [1] to [3], in which the neurokinin B signal blocker is an expression inhibitor of neurokinin B (NKB), a neurokinin 3 receptor, a tachykinin processing enzyme, gastrin-releasing peptide (GRP), or a GRP receptor.
[17] The prophylactic or therapeutic agent according to any one of [1] to [3], in which the neurokinin B signal blocker is a removing agent for neurons expressing a neurokinin 3 receptor or a GRP receptor.
[18] The prophylactic or therapeutic agent according to [17], in which the removing agent is GRP, a specific binding substance to a GRP receptor, NKB, or a specific binding substance to a neurokinin 3 receptor, to which a cytotoxic substance is bound.
[19] The prophylactic or therapeutic agent according to [18], in which the cytotoxic substance is a ribosome-inactivating protein or a diphtheria toxin.
[20] The prophylactic or therapeutic agent according to [19], in which the ribosome-inactivating protein is saporin, ricin, or abrin.
[21] A prophylactic or therapeutic medicinal composition for an IL-31-mediated disease comprising the prophylactic or therapeutic agent according to any one of [1] to [20] and a pharmaceutically acceptable carrier.
[22] A prophylactic or therapeutic medicinal composition for atopic dermatitis comprising the prophylactic or therapeutic agent according to any one of [1] to [20] and a pharmaceutically acceptable carrier.

### Advantageous Effects of Invention

According to the present invention, it is possible to provide a prophylactic or therapeutic technique for an IL-31-mediated disease such as atopic dermatitis.

### Brief Description of Drawings

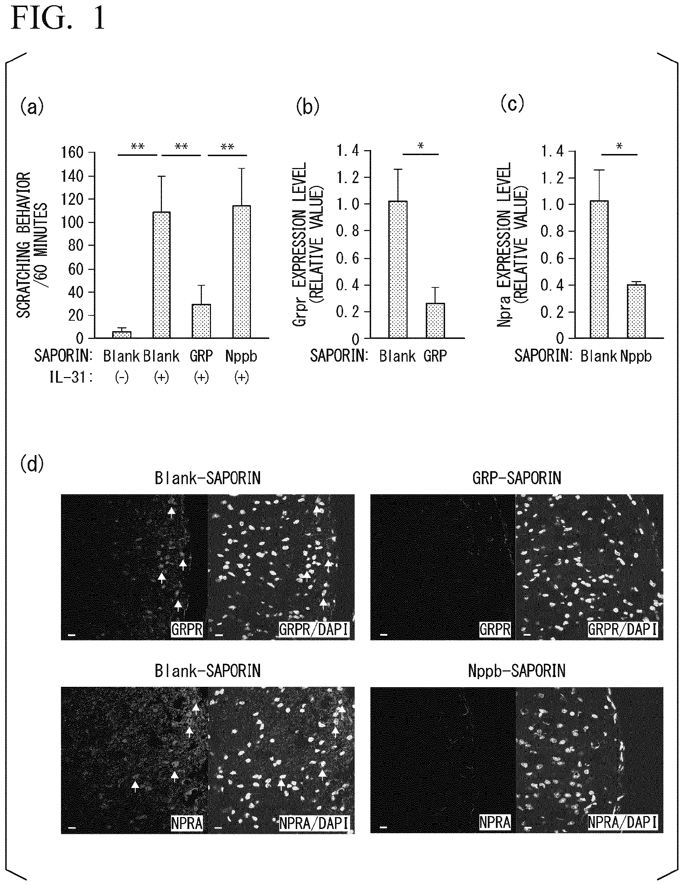

Fig. 1(a) shows a result obtained from injecting each of gastrin-releasing peptide (GRP)-saporin or natriuretic polypeptide b (Nppb)-saporin into medullary cavities of wild-type C57BL/6 mice, administering IL-31 to these mice, and then analyzing scratching behavior of these mice in Experimental Example 1; Fig. 1(b) shows a result obtained from injecting GRP-saporin into the medullary cavities of wild-type C57BL/6 mice and analyzing an expression level of GRPR mRNA in a spinal cord in Experimental Example 1; Fig. 1(c) shows a result obtained from injecting Nppb-saporin into the medullary cavities of wild-type C57BL/6 mice and analyzing an expression level of mRNA of NPRA in a spinal cord in Experimental Example 1; and Fig. 1(d) shows a result obtained from injecting GRP-saporin or Nppb-saporin into the medullary cavities of wild-type C57BL/6 mice, excising the spinal cord, and subjecting it to immunostaining with anti-GRPR antibodies and anti-NPRA antibodies in Experimental Example 1.

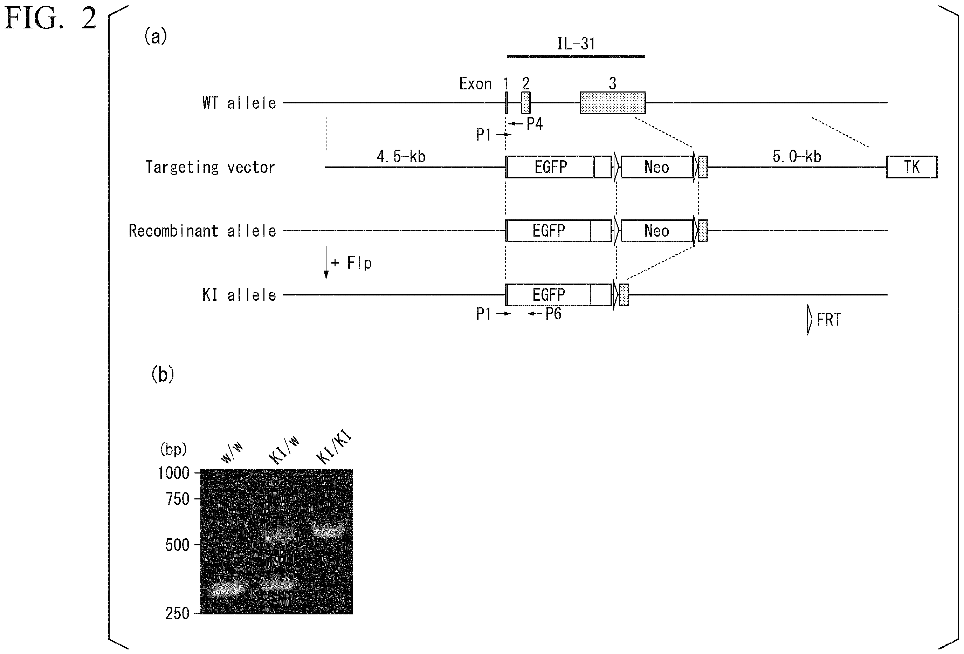

Fig. 2(a) shows a structure of an IL-31 gene of wild-type C57BL/6 mice, a structure of a targeting vector, a gene structure after insertion of a neo cassette by homologous recombination, and a structure after removal of a neo cassette by a flippase in Experimental Example 2; and Fig. 2(b) is a figure showing that each PCR product has been electrophoresed on agarose gel of 2% in Experimental Example 2, in which the length of the PCR product using a wild-type C57BL/6 mouse-derived genome as a template is 300 bp, and the length of the PCR product using a knock-in mouse-derived genome as a template is 550 base pairs.

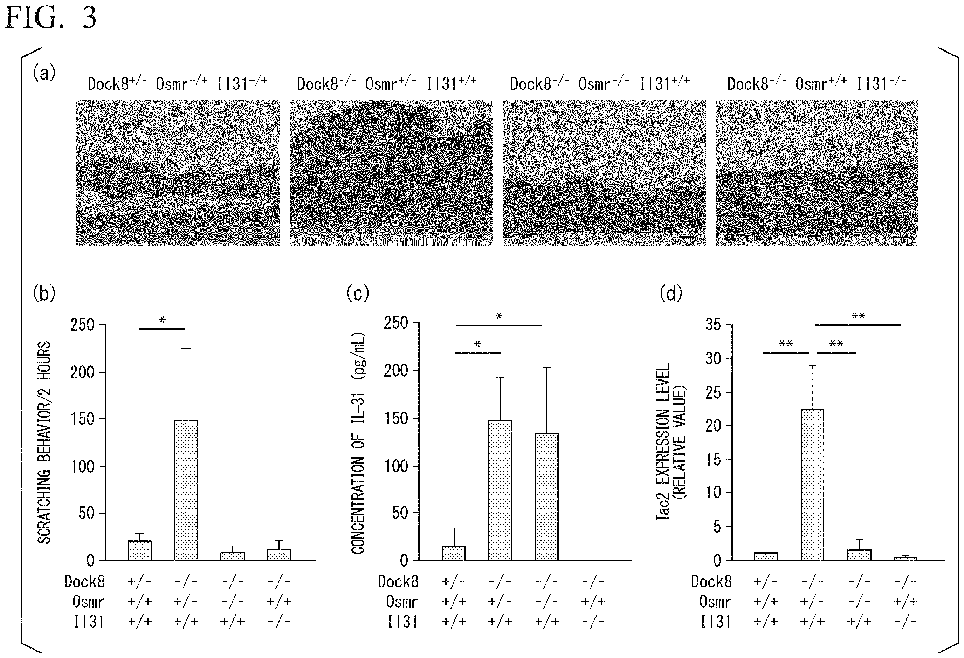

Fig. 3(a) is a figure showing analysis of skin tissues of mice in each genotype by hematoxylin staining in Experimental Example 3; Fig. 3(b) is a figure showing analysis of scratching behavior of mice in each genotype in Experimental Example 3; Fig. 3(c) shows a result obtained from analyzing a concentration of IL-31 in a serum of mice in each genotype in Experimental Example 3; and Fig. 3(d) shows a result obtained from analyzing an expression level of Tac2 mRNA in dorsal root ganglion (DRG) of mice in each genotype in Experimental Example 3.

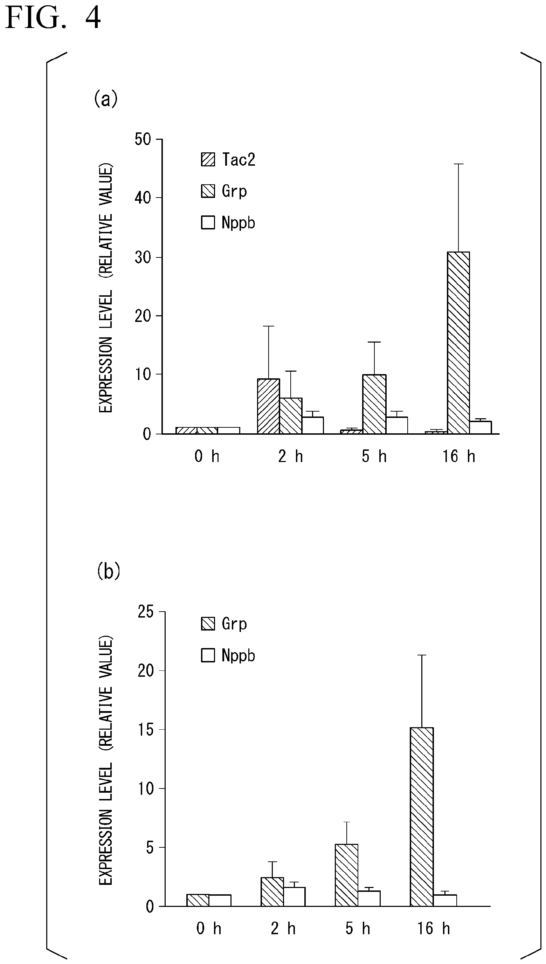

Fig. 4(a) shows a result obtained from analyzing an expression level of mRNA in each gene after stimulating DRG neurons with IL-31 in Experimental Example 4; and Fig. 4(b) shows a result obtained from analyzing an expression level of each gene after stimulating DRG neurons with NKB in Experimental Example 4.

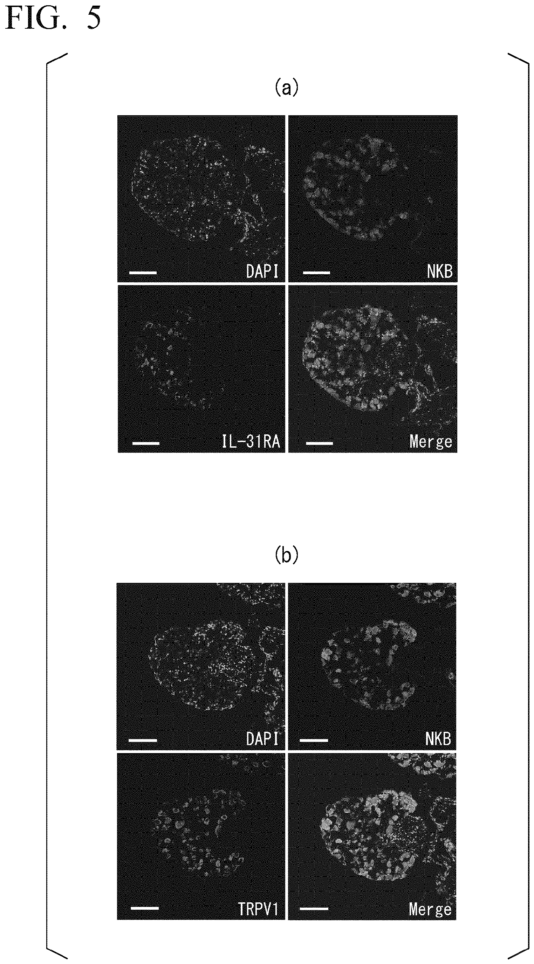

Fig. 5 is a result obtained from subjecting DRG of Dock8^{4-/-} AND Tg mice to immunostaining with anti-NKB antibodies and anti-IL-31RA antibodies in Experimental Example 5; and Fig. 5(b) is a result obtained from subjecting the DRG of the Dock8^{-/-} AND Tg mice to immunostaining with anti-NKB antibodies and anti-TRPVl antibodies in Experimental Example 5.

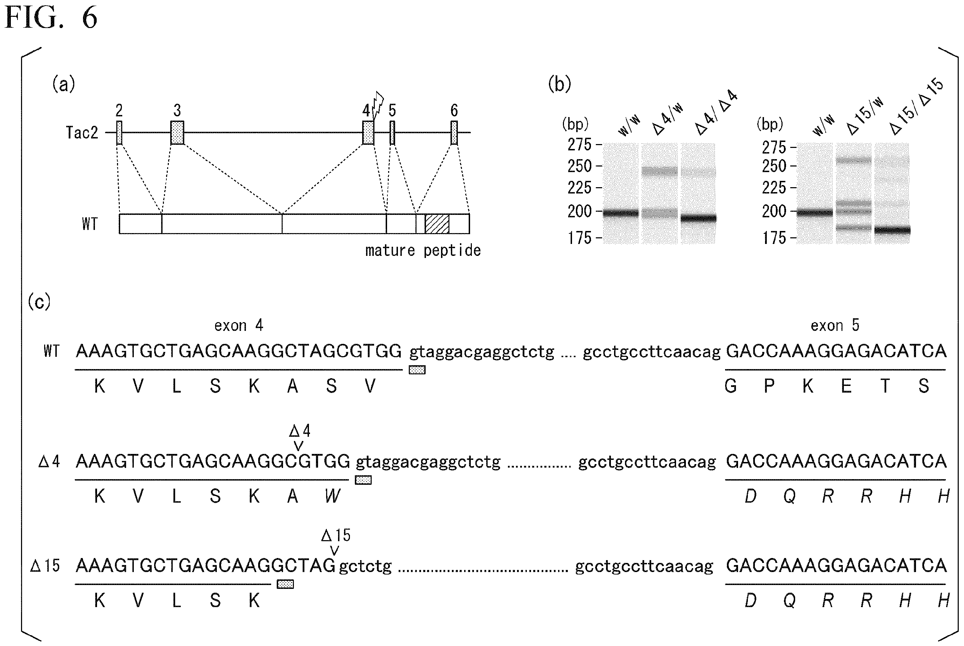

Fig. 6(a) is a diagram figure (or result) schematically showing a mouse Tac2 gene and an NKB protein encoded by the mouse Tac2 gene in Experimental Example 6; Fig. 6(b) is a figure showing identification of a Tac2 genotype of Tac2 mutant mice Δ4 and Δ15 by PCR and electrophoresis in Experimental Example 6; and Fig. 6(c) is a figure showing genomic sequences of two mutations and amino acid sequences encoded by the transcribed mRNA in Experimental Example 6.

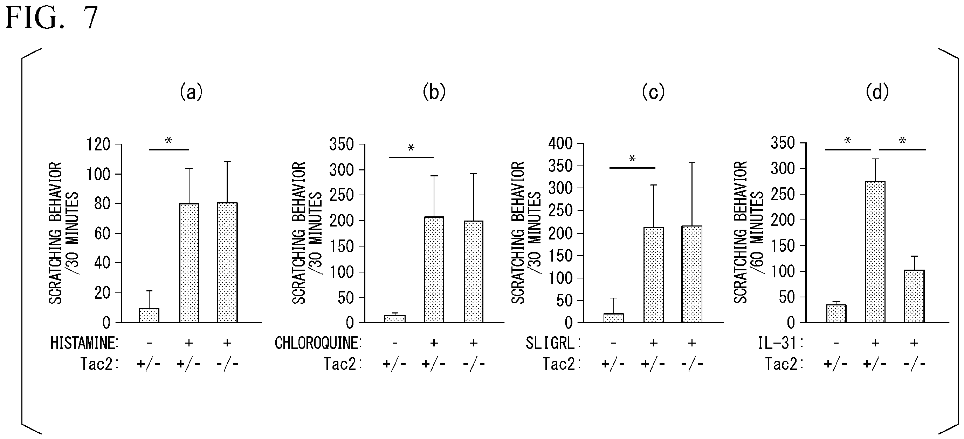

Fig. 7(a) shows a result obtained from analyzing scratching behavior after administration of histamine to Tac2^{-/-} (Δ4) mice in Experimental Example 7; Fig. 7(b) shows a result obtained from analyzing scratching behavior after administration of chloroquine to Tac2^{-/-} (Δ4) mice in Experimental Example 7; Fig. 7(c) shows a result obtained from analyzing scratching behavior after administration of PAR2 agonist to Tac2^{-/-} (Δ4) mice in Experimental Example 7; and Fig. 7(d) shows a result obtained from analyzing scratching behavior after administration of IL-31 to Tac2^{-/-} (Δ4) mice in Experimental Example 7.

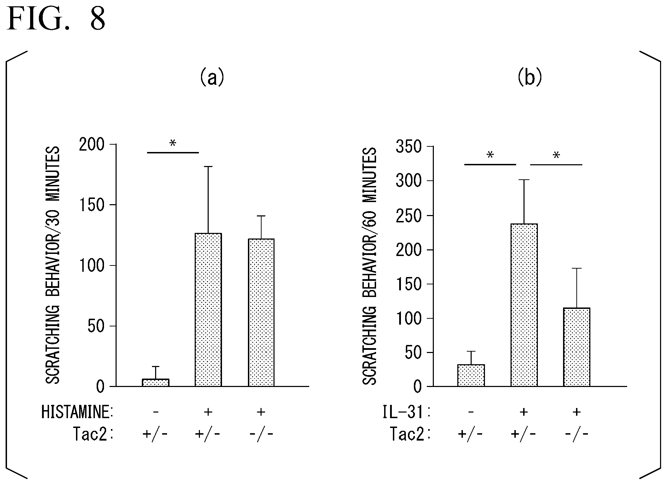

Fig. 8(a) shows a result obtained from analyzing scratching behavior after administration of histamine to Tac2^{-/-} (Δ15) mice in Experimental Example 7; and Fig. 8(b) shows a result obtained from analyzing scratching behavior after administration of IL-31 to Tac2^{-/-} (Δ15) mice in Experimental Example 7.

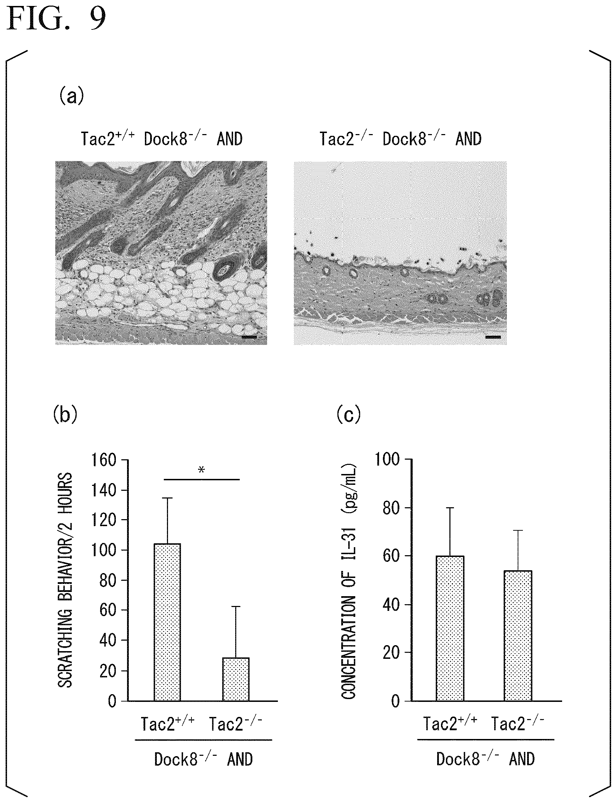

Fig. 9 shows comparison results, with Fig. 9(a) showing skin tissues, Fig. 9(b) showing scratching behavior, and Fig. 9(c) showing an IL-31 level in a serum in a case where Dock8^{-/-} AND Tg mice in which Tac is expressed or Tac is not expressed (Tac2^{-/-} (Δ4)) are established in Experimental Example 7.

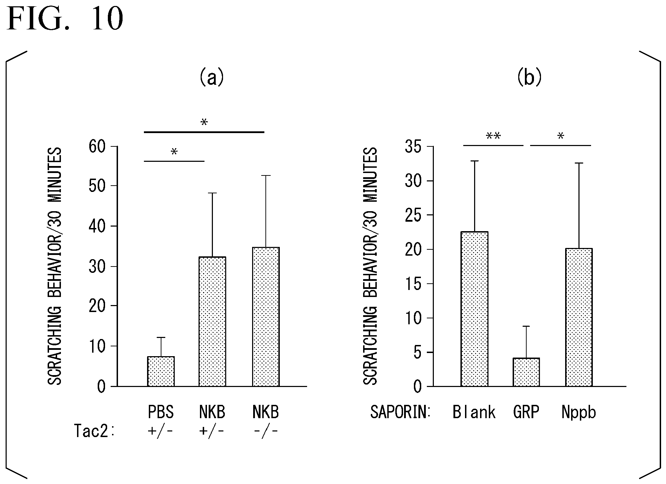

Fig. 10(a) shows a result obtained from injecting NKB into medullary cavities of Tac2^{-/-} (Δ4) mice and analyzing scratching behavior in Experimental Example 8; and Fig. 10(b) shows a result obtained from injecting GRP-saporin or Nppb-saporin to remove neurons expressing GRPR or NPRA, then injecting NKB into a medullary cavity, and then analyzing scratching behavior in Experimental Example 8.

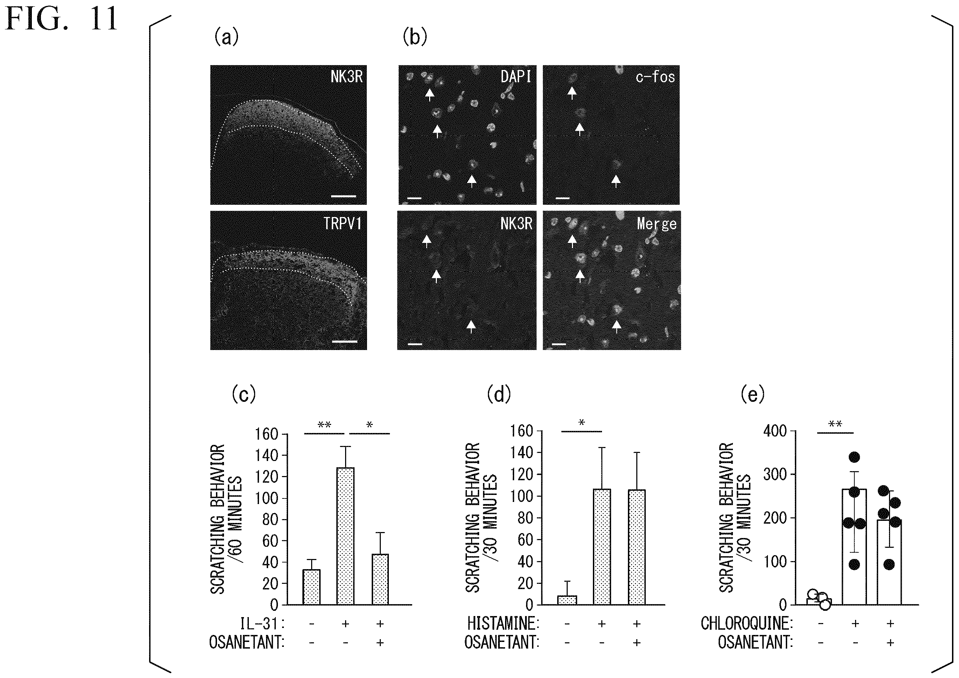

Fig. 11(a) shows a result obtained from subjecting a spinal cord dorsal horn to immunostaining with anti-NK3R antibodies and anti-TRPV1 antibodies in Experimental Example 9; Fig. 11(b) shows a result obtained from administering IL-31 to C57BL/6 mice and then subjecting them to immunostaining with anti-NK3R antibodies and anti-c-fos antibodies in Experimental Example 9; Fig. 11(c) shows a result obtained from injecting osanetant, administering IL-31, and then analyzing scratching behavior in Experimental Example 9; Fig. 11(d) shows a result obtained from injecting osanetant, administering histamine, and then analyzing scratching behavior in Experimental Example 9; and Fig. 11(e) shows a result obtained from injecting osanetant, administering chloroquine, and then analyzing scratching behavior in Experimental Example 9, in which white circles and black circles in the figure are plots of measured values in each mouse.

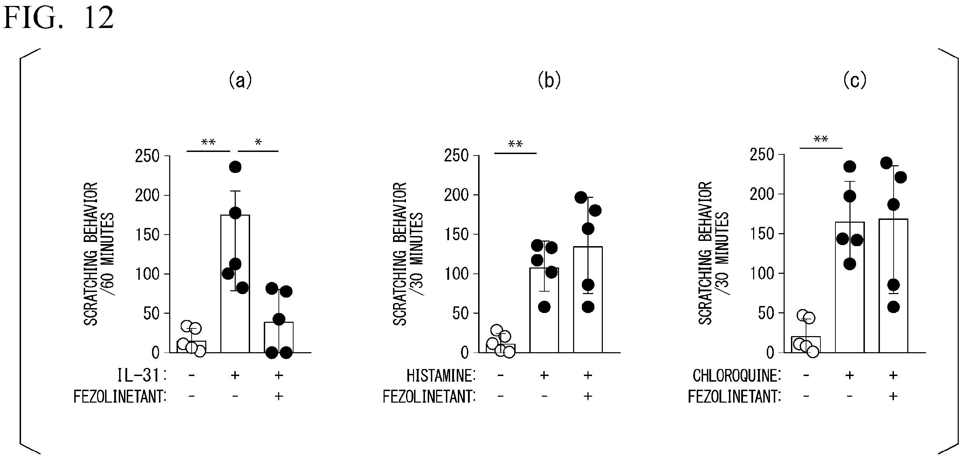

Fig. 12(a) shows a result obtained from administering fezolinetant, administering IL-31, and then analyzing scratching behavior in Experimental Example 9; Fig. 12(b) shows a result obtained from administering fezolinetant, administering histamine, and then analyzing scratching behavior in Experimental Example 9; and Fig. 12(c) shows a result obtained from administering fezolinetant, administering chloroquine, and then analyzing scratching behavior in Experimental Example 9, in which white circles and black circles in the figure are plots of measured values in each mouse.

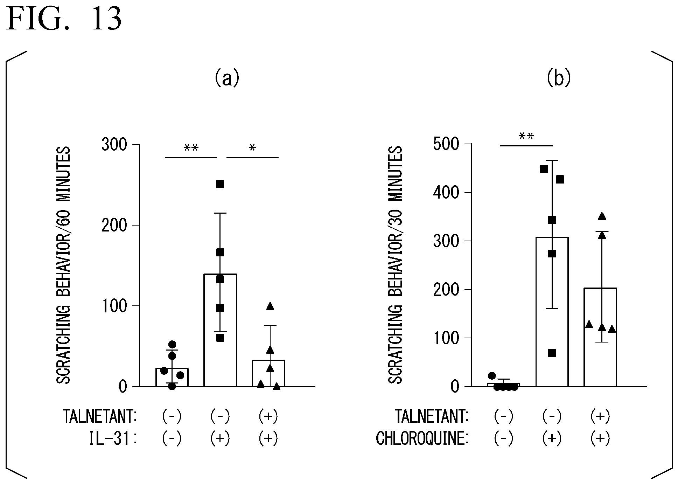

Fig. 13(a) shows a result obtained from administering talnetant, administering IL-31, and then analyzing scratching behavior in Experimental Example 9; and Fig. 13(b) shows a result obtained from administering talnetant, administering chloroquine, and then analyzing scratching behavior in Experimental Example 9, in which black circles, black squares, and black triangles in the figure are plots of measured values in each mouse.

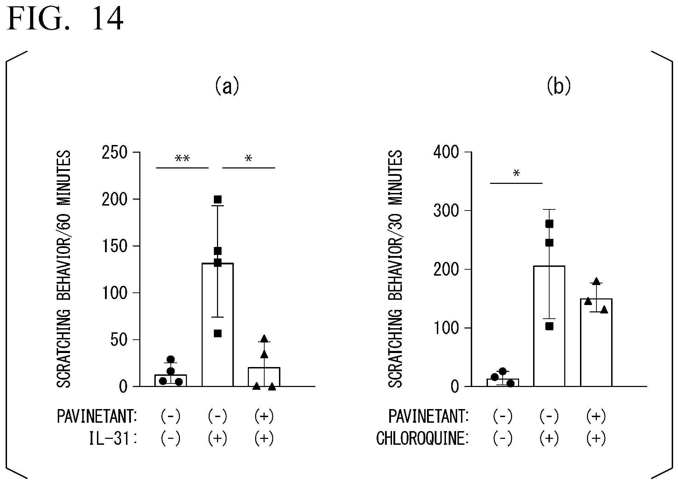

Fig. 14(a) shows a result obtained from administering pavinetant, administering IL-31, and then analyzing scratching behavior in Experimental Example 9; and Fig. 14(b) shows a result obtained from administering pavinetant, administering chloroquine, and then analyzing scratching behavior in Experimental Example 9, in which black circles, black squares, and black triangles in the figure are plots of measured values in each mouse.

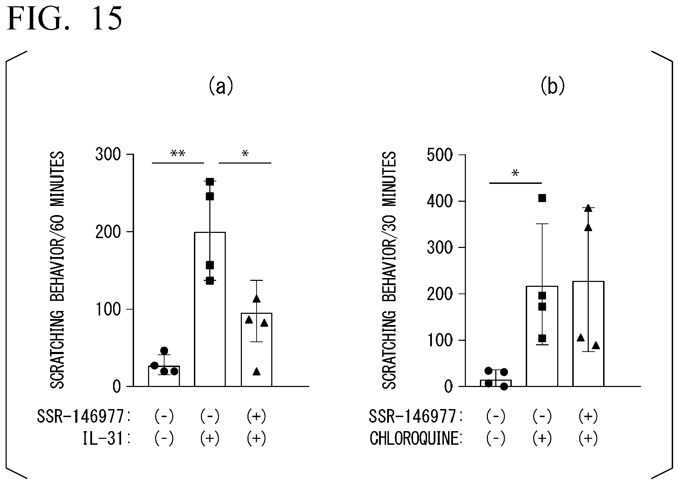

Fig. 15(a) shows a result obtained from administering SSR-146977, administering IL-31, and then analyzing scratching behavior in Experimental Example 9; and Fig. 15(b) shows a result obtained from administering SSR-146977, administering chloroquine, and then analyzing scratching behavior in Experimental Example 9, in which black circles, black squares, and black triangles in the figure are plots of measured values in each mouse.

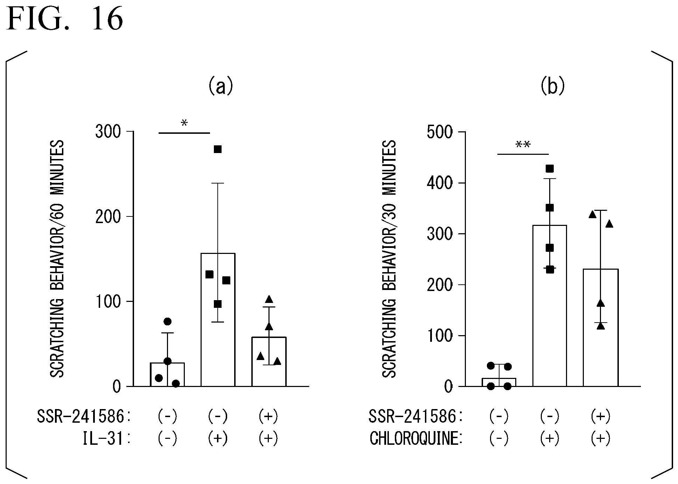

Fig. 16(a) shows a result obtained from administering SSR-241586, administering IL-31, and then analyzing scratching behavior in Experimental Example 9; and Fig. 16(b) shows a result obtained from administering SSR-241586, administering chloroquine, and then analyzing scratching behavior in Experimental Example 9, in which black circles, black squares, and black triangles in the figure are plots of measured values in each mouse.

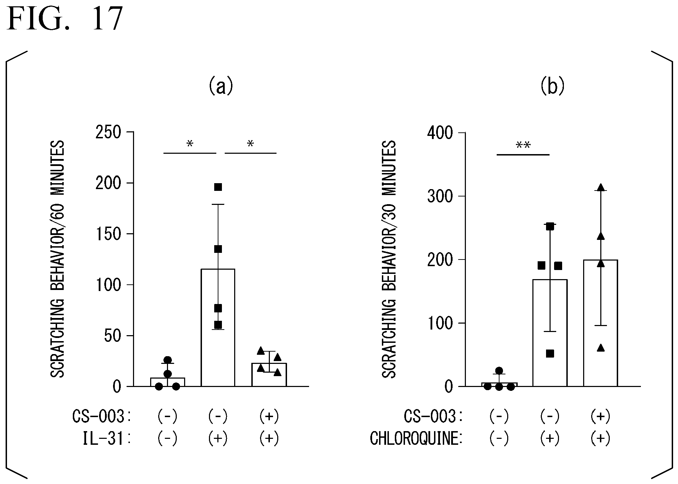

Fig. 17(a) shows a result obtained from administering CS-003, administering IL-31, and then analyzing scratching behavior in Experimental Example 9; and Fig. 17(b) shows a result obtained from administering CS-003, administering chloroquine, and then analyzing scratching behavior in Experimental Example 9, in which black circles, black squares, and black triangles in the figure are plots of measured values in each mouse.

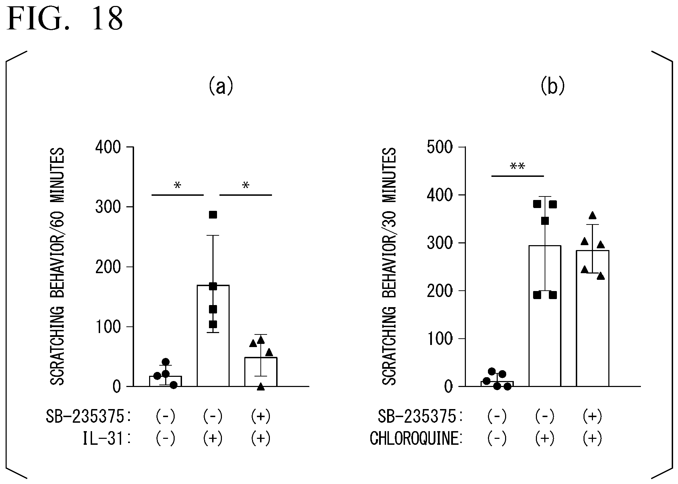

Fig. 18(a) shows a result obtained from administering SB-235375, administering IL-31, and then analyzing scratching behavior in Experimental Example 9; and Fig. 18(b) shows a result obtained from administering SB-235375, administering chloroquine, and then analyzing scratching behavior in Experimental Example 9, in which black circles, black squares, and black triangles in the figure are plots of measured values in each mouse.

### Description of Embodiments

### [Prophylactic or Therapeutic Agent]

In one embodiment of the present invention, a prophylactic or therapeutic agent including a neurokinin B signal blocker for an IL-31-mediated disease is provided. In one embodiment of the present invention, the prophylactic or therapeutic agent for an IL-31-mediated disease including a neurokinin B signal blocker as an active ingredient may be provided.

### (1L-31-Mediated Disease)

In the present invention, an IL-31-mediated disease is a disease in which IL-31 is involved in the onset or progression. The term "IL-31-mediated" indicates not only that IL-31 is involved in the onset or progression of a disease through direct action, but also that, as a result of the action of IL-31 on body tissues or somatic cells, new biomolecules are produced, expressed, or released or activated, so that a disease develops or progresses due to the biomolecules.

Examples of the IL-31-mediated disease include atopic dermatitis, pruritus in atopic dermatitis, dermatomyositis, pruritus in dermatomyositis, chronic dermatitis, allergic contact dermatitis, dermatitis herpetiformis, pruritus in cutaneous T cell lymphoma, prurigo nodularis, prurigo chronica multiformis, urticaria pigmentosa, bullous pemphigoid, and the like, but are not limited thereto. In these diseases, it has been reported that the expression of IL-31 and IL-31 receptors in a lesioned tissue is enhanced, a serum IL-31 level is higher than that in healthy subjects, and these phenomena correlate with the onset of a disease.

It is possible to prevent or treat the IL-31-mediated disease or symptoms associated with the disease by suppressing the IL-31 production in vivo or by inhibiting the action of IL-31 after the IL-31 production. IL-31 exerts its activity through the binding of IL-31 to its receptor, intracellularly transmitting the stimulation signal. Therefore, the action of IL-31 can be suppressed by blocking the binding of IL-31 to its receptor. For that purpose, for example, an anti-IL-31 antibody or an anti-IL-31 receptor antibody can be used. A heterodimer consisted of an IL-31 receptor A and an oncostatin M receptor is formed to function as the IL-31 receptor, and it has been reported that an anti-IL-31 receptor A antibody is used to suppress atopic dermatitis and pruritus in atopic dermatitis (Kabashima K, et al. Nemolizumab in patients with moderate-to-severe atopic dermatitis: Randomized, phase II, long-term extension study. J Allergy Clin Immunol. 142:1121-1130, 2018).

Suppression of the action of IL-31 is also achieved by inhibiting the production, expression or release, or activation of biomolecules involved in the IL-31-mediated disease, induced by the stimulation of the IL-31 receptor. Such activation includes a case where inactive or low-activity biomolecules are activated or highly activated to enhance the function of the biomolecules.

### (Neurokinin B Signal)

So far, the present inventors have clarified that Dock 8-/- AND Tg mice show enhanced scratching behavior, and a concentration of IL-31 in blood is increased. In addition, as detailed in examples, the present inventors have clarified that an expression level of Tachykinin2 (Tac2) gene increases by a factor of about 23 in dorsal root ganglia of these mice.

The Tac2 gene encodes preprotachykinin-3 (PPT-3) that is a precursor of a neuropeptide. PPT-3 is post-translationally modified by a processing enzyme, and then becomes mature neurokinin B (NKB). NKB binds to a neurokinin receptor 3 (NK3R).

The present inventors have clarified that an expression level of NKB increases as the concentration of IL-31 increases, NKB activates neurons expressing NK3R, neurons expressing gastrin-releasing peptide (GRP) are activated, and neurons expressing GRP receptors are activated, so that scratching behavior of mice is induced.

In the present specification, molecules and neural circuits that function in a process of NKB inducing scratching behavior are referred to as a neurokinin B signal.

The scratching behavior induced by the increased concentration of IL-31 can be suppressed by blocking neurokinin B signal described above. In addition, the 1L-31-mediated disease can be prevented or treated by blocking neurokinin B signal.

The neurokinin B signal blocker may be an agent that suppresses functions of PPT-3, a processing enzyme, NKB, NK3R, GRP, GRP receptors, and the like.

The neurokinin B signal blocker may be an agent that suppresses expression levels of PPT-3, a processing enzyme, NKB, NK3R, GRP, GRP receptors, and the like, or an agent that removes neurons expressing NK3R receptors or GRP receptors.

### (Antagonist)

The neurokinin B signal blocker may be an antagonist (antagonistic drug) of NK3R. The neurokinin B signal blocker can also be referred to as an NK3R signal blocker. The NK3R antagonist can also be referred to as an NK3R blocker (blocking drug).

NKB has the highest affinity for NK3R, and NK3R is activated mainly by the binding of NKB.

As will be described later in examples, the present inventors have clarified that the administration of IL-31 increases the expression of NKB to induce scratching behavior. The inventors have also clarified that this scratching behavior is suppressed by the NK3R antagonists such as osanetant, fezolinetant, talnetant, pavinetant, SSR-146977, SB-235375, SSR-241586, and CS-003.

That is, the scratching behavior induced by an increased concentration of IL-31 can be suppressed by inhibiting the activation of NK3R. In addition, the 1L-31-mediated disease can be prevented or treated by inhibiting the activation of NK3R.

Factors that inhibit the activation of NK3R include low molecular weight compounds, peptides, an antibody against NK3R, and the like.

In one embodiment of the present invention, in the prophylactic or therapeutic agent for an IL-31 -mediated disease, the above described NK3R antagonist is a compound represented by Formula (1), (2), or (3) or a salt thereof, or a solvate thereof.

In Formula (1), an alkyl group or an alkoxy group may be linear or branched. A halogen atom is a fluorine atom, a chlorine atom, a bromine atom, or an iodine atom, and is preferably a fluorine atom, a chlorine atom, or an iodine atom.

An acyl group may be a formyl group or an alkylcarbonyl group having 1 to 6 carbon atoms.

The salt of the compound represented by Formula (1), (2), or (3) is an inorganic acid salt or an organic acid salt, and exemplary examples thereof include salts that are suitable for separation or crystallization of the compound represented by Formula (1), (2), or (3). More specifically, exemplary examples of the salt of the compound represented by Formula (1) include a picrate and an oxal salt; a salt with an optically active acid, for example, mandelate and camphor sulfonate; and a pharmaceutically acceptable salt.

Exemplary examples of the pharmaceutically acceptable salt of the compound represented by Formula (1), (2), or (3) include a hydrochloride, a bromate, a sulfate, a hydrogen sulfate, a dihydrogen phosphate, a methanesulfonate, a methylsulfate, a maleate, a fumarate, a 2-naphthalene sulfonate, a benzenesulfonate, a glycolate, a gluconate, a citrate, an isethionate, a para-toluenesulfonate, and the like.

The compound represented by Formula (1), (2), or (3) may be a racemate or a pure enantiomer.

The compound represented by Formula (1) may be osanetant. Osanetant is the international nonproprietary name (INN) of N-[1-[3-[(3R)-1-benzoyl-3-(3,4-dichlorophenyl)piperidine-3-yl)propyl)-4-phenylpiperidine-4-yl]-N-methyl acetamide, and is a compound having a chemical structure represented by Formula (4). The CAS number of the compound of Formula (4) is 160492-56-8.

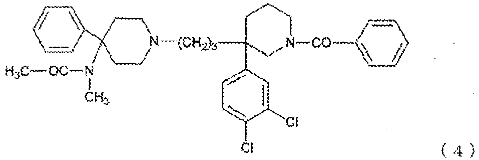

The compound represented by Formula (1) may be SSR-146977. SSR-146977 is N-[1-[3-[1-benzoyl-3 (R)-(3,4-dichlorophenyl)piperidine-3-yl]propyl]-4-phenylpiperidine-4-yl]-N',N'-dimethylurea, and is a compound having a chemical structure represented by Formula (5). The CAS number of the compound of Formula (5) is 264618-44-2.

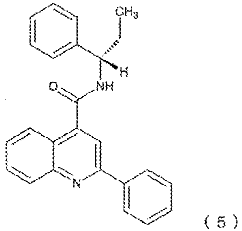

The compound represented by Formula (1) may be SSR-241586. SSR-241586 is (+)-1'-[2-[4-benzoyl-2(R)-(3,4-dichlorophenyl)morpholine-2-yl]ethyl]-N,N-dimethyl-1,4'-bipiperidin-4'-carboxamide, and is a compound having a chemical structure represented by Formula (6). The CAS number of the compound of Formula (6) is 1239279-30-1.

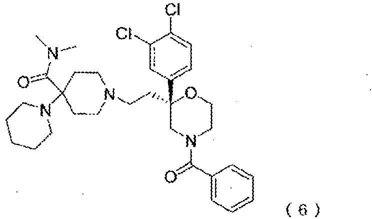

The compound represented by Formula (1) may be CS-003. CS-003 is 1'-[2-[2-(R)-(3,4-dichlorophenyl)-4-(3,4,5-)trimethoxybenzoyl]morpholine-2-yl] ethyl]spiro[benzo[c]thiophene-1(3H)-4'-piperidine]2(S)-oxide, and is a compound having a chemical structure represented by Formula (7). The CAS number of the compound of Formula (7) is 191672-52-3.

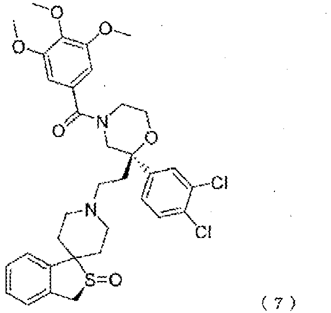

The compound represented by Formula (2) may be talnetant. Talnetant is N-(α-ethylbenzyl)-3-hydroxy-2-phenylquinoline-4-carboxamide, and is a compound having a chemical structure represented by Formula (8). The CAS number of the compound represented by Formula (8) is 174636-32-9.

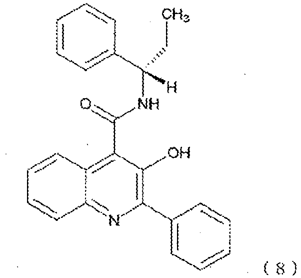

The compound represented by Formula (2) may be pavinetant. Pavinetant is 3-(methanesulfonamide)-2-phenyl-N-[1S]-1-phenylpropyl]quinoline-4-carboxamide, and is a compound represented by Formula (9). The CAS number of the compound represented by Formula (9) is 941690-55-7.

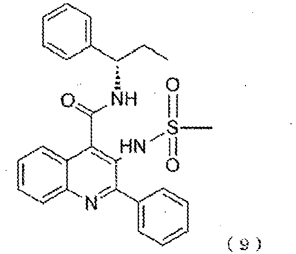

The compound represented by Formula (2) may be SB-235375. SB-235375 is 2-[2-phenyl-4-[N-[1(S)-phenylpropyl]carbamoyl]quinoline-3-yloxy]acetic acid, and is a compound represented by Formula (10). The CAS number of the compound represented by Formula (10) is 224961-34-6.

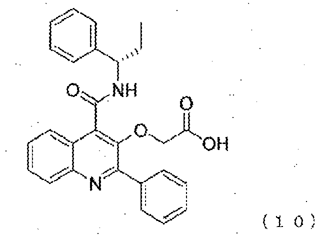

The compound represented by Formula (3) may be fezolinetant. Fezolinetant is (4-fluofophenyl)-[(8R)-8-methyl-3-(3-methyl-1,2,4-thiadiazole-5-yl)-6,8-dihydfo-5H-[1,2,4]triazolo[4,3-a]pyrazine-7-yl]-methanone, and is a compound represented by Formula (11). The CAS number of the compound represented by Formula (11) is 1629229-37-3.

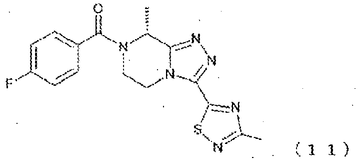

### (Tachykinin Processing Enzyme)

A mouse Tac2 gene encodes preprotachykinin-3 (PPT-3). PPT-3 is post-translationally modified, and then neurokinin B (NKB) is synthesized.

It is considered that this post-translational modification proceeds as follows. The precursor PPT-3 is cleaved at a position where two consecutive lysine residues or arginine residues are present. This cleavage is catalyzed by a prohormone converterase (PC).

PCs constitute a proprotein convertase subtilisin/kexin (PCSK) family. The PCSK family is composed of PCSK1 to -9. Among these, PCSK1 and PCSK2 are major enzymes that process in vivo peptides.

In neural tissues of mice, in which expression levels of PCSK1 and PCSK2 are decreased, a concentration of neurokinin A is decreased.

The lysine residue and arginine residue at the C-terminus of the cleaved peptide are removed by carboxypeptidase. Among these, an enzyme mainly involved in this reaction is carboxypeptidase E (CPE).

A peptide from which a basic peptide has been removed contains a glycine residue at the C-terminus. An α-position amino group of this C-terminus glycine residue is amidated by a peptidyl-glycine alpha-amidating monooxygenase (PAM).

In humans, PAM is encoded by a PAM gene. The PAM is composed of a peptidylglycine alpha-hydroxylating monooxygenase (PHM) domain and a peptidyl-alpha-hydroxygycine alpha-amidating lyase (PAL) domain.

The PHM domain hydroxylates the glycine residue at the C-terminus of a peptide. The PAL domain subsequently removes the hydroxylated glycine residue. With a series of reactions, glycine is cleaved, and the C-terminus of a peptide is amidated.

Human PPT-3 and mouse PPT-3 have an amino acid sequence of NKB shown in SEQ ID NO: 2. In addition, human PPT-3 and mouse PPT-3 have the above described basic amino acid residue and glycine residue involved in processing.

Thus, it is considered that post-translational modifications of human PPT-3 and mouse PPT-3 proceed by the same mechanism.

Cleavage with PCSK and CPE, and amidation with PAM are required for NKB to have activity. That is, the action of NKB can be suppressed by inhibiting the activity of these enzymes.

The neurokinin B signal blocker is an inhibitor of a tachykinin processing enzyme or a decomposition accelerator of a tachykinin processing enzyme. The inhibitor of a tachykinin processing enzyme or the decomposition accelerator of a tachykinin processing enzyme can suppress the action of NKB to suppress the scratching behavior induced by an increased concentration of 1L-31. Therefore, the inhibitor of a tachykinin processing enzyme can be used to prevent or treat the IL-31-mediated disease.

Exemplary examples of tachykinin processing enzymes include proprotein convertase subtilisin/kexin 1 (PCSK1), proprotein convertase subtilisin/kexin 2 (PCSK2), carboxypeptidase E (CPE), peptidyl-glycine alpha-amidating monooxygenase (PAM), and the like, but are not limited thereto, and any enzyme may be used as long as the enzyme is involved in the processing of tachykinin.

The inhibitor of the tachykinin processing enzyme may be a low molecular weight compound, may be a peptide, or may be an antibody against the tachykinin processing enzyme, or the like.

### (Expression Inhibitor)

In one embodiment of the present invention, the prophylactic or therapeutic agent for an IL-31-mediated disease, in which the neurokinin B signal blocker is an expression inhibitor of neurokinin B, a neurokinin 3 receptor, a tachykinin processing enzyme, GRP, or a GRP receptor, is provided.

Exemplary examples of the expression inhibitor include siRNA, shRNA, and the like. An expression inhibitor for a gene encoding a protein involved in the NKB signal is administered to reduce the expression level of any of the above factors, so that the NKB signal can be blocked. As a result, the scratching behavior induced by an increased concentration of IL-31 can be suppressed. In addition, the IL-31-mediated disease can be prevented or treated by administering these expression inhibitors.

### (Denervation of Neurons)

In one embodiment of the present invention, the prophylactic or therapeutic agent for an IL-31-mediated disease as a removing agent for neurons expressing a neurokinin 3 receptor or a GRP receptor is provided.

The removing agent for neurons may be GRP, a specific binding substance to a GRP receptor, NKB, or a specific binding substance to a neurokinin 3 receptor, to which a cytotoxic substance is bound.

Examples of a cytotoxic substance include a ribosome-inactivating protein (RIP), a diphtheria toxin, and the like. Exemplary examples of the RIP include saporin, ricin, abrin, and the like.

The GRP bound to a cytotoxic substance is captured by GRP receptor-expressing neurons and taken up into cells by endocytosis. Similarly, neurokinin B bound to a cytotoxic substance is captured by neurokinin 3 receptor (NK3R)-expressing neurons and taken up into cells by endocytosis. Subsequently, the neurons that have taken up the cytotoxic substance are killed and denervated.

As will be described later in examples, the present inventors denervated the neurons by administering GRP-saporin obtained by conjugating saporin to GRP to mice to induce cell death in neurons expressing the GRP receptor. As a result, it was clarified that the scratching behavior induced by the administration of IL-31 and NKB was suppressed in the mice from which the neurons expressing the GRP receptor were denervated.

That is, the neurokinin B signal can be blocked by denervating the neurons expressing the GRP receptor or NK3R. As a result, the scratching behavior induced by an increased concentration of IL-31 can be suppressed.

In addition, the above described removing agent for neurons may be an agent in which a cytotoxic substance is bound to proteins specifically captured on surfaces of target neurons. The proteins captured on the surfaces of the target neurons may be, for example, ligands bound to a receptor that is specifically expressed on the target neurons, or a specific binding substance expressed on the target neurons and specifically bound to proteins that are present on cell surfaces. Exemplary examples of the specific binding substances include antibodies, antibody fragments, aptamers, and the like. Exemplary examples of the antibodies include an anti-GRP receptor antibody, an anti-NK3R antibody, and the like.

### [Medicinal Composition]

In one embodiment of the present invention, a prophylactic or therapeutic medicinal composition for an IL-31-mediated disease including the above described prophylactic or therapeutic agent for an IL-31-mediated disease and a pharmaceutically acceptable carrier is provided.

The medicinal composition of the present embodiment may be in a dosage form used orally or in a dosage form used parenterally. Exemplary examples of the dosage form used orally include tablets, capsules, elixirs, microcapsules, and the like. Exemplary examples of the dosage form used parenterally include injections, inhalants, suppositories, topical skin preparations, patches, and the like.

Exemplary examples of the pharmaceutically acceptable carrier include solvents such as sterilized water and physiological saline; binders such as gelatin, cornstarch, tragant gum, and arabic rubber; excipients such as crystalline cellulose; and swelling agents such as alginic acid.

The medicinal composition may further contain additives. Exemplary examples of the additives include lubricants such as magnesium stearate; sweeteners such as sucrose, lactose, and saccharin; flavors such as peppermint and *Gaultheria adenothrix* oil; stabilizers of benzyl alcohol and phenol; buffers such as phosphate and sodium acetate; dissolution adjuvants such as benzyl benzoate and benzyl alcohol; antioxidants; and preservatives.

The medicinal composition can be formulated by appropriately combining and mixing the above carriers and additives in a unit dose form required for generally accepted pharmaceutical practice.

Administration methods to patients known to those skilled in the art, such as intramedullary injection, intraarterial injection, intravenous injection, subcutaneous injection, as well as intranasal, transbronchial, intramuscular, percutaneous, or oral administration, can be adopted. A dose varies depending on the weight and age of a patient, the patient's symptoms, an administration method, and the like, but those skilled in the art can appropriately select an appropriate dose.

A dose of the prophylactic or therapeutic agent for an IL-31-mediated disease such as atopic dermatitis varies depending on the type of the prophylactic or therapeutic agent for atopic dermatitis, the patient's symptoms, or the like. However, in a case of oral administration, it is considered that in general adults (assuming a weight of 60 kg), a dose of about 0.1 to 500 mg per day, preferably about 1.0 to 250 mg per day, or more preferably about 1.0 to 100 mg per day is appropriately administered once a day or in several divided doses throughout a day.

In a case of parenteral administration, a dose thereof varies depending on the type of the prophylactic or therapeutic agent for an IL-31-mediated disease such as atopic dermatitis, the patient's symptoms, an administration site, an administration method, or the like. However, in a case of systemic administration, it is considered that in general adults (assuming a weight of 60 kg), a dose of about 0.1 to 500 mg per day, preferably about 1.0 to 250 mg per day, or more preferably about 1.0 to 100 mg per day is appropriately administered once a day or in several divided doses throughout a day. In a case of topical administration, it is considered that in general adults (assuming a weight of 60 kg), a dose of about 0.001 to 10 mg per day, preferably about 0.01 to 5 mg per day, or more preferably about 0.02 to 2 mg per day is appropriately administered once a day or in several divided doses throughout a day.

### [Other Embodiments]

In one embodiment of the present invention, a prophylactic or therapeutic method for an IL-31-mediated disease including administration of an effective amount of a neurokinin B signal blocker to a patient in need of treatment is provided.

In one embodiment of the present invention, a neurokinin B signal blocker for prevention or treatment of an IL-31-mediated disease is provided.

In one embodiment of the present invention, a neurokinin B signal blocker for producing a prophylactic or therapeutic agent for an IL-31-mediated disease is provided.

In each of the above embodiments, the neurokinin B signal blocker may be a neurokinin 3 receptor antagonist. The neurokinin B signal blocker is an inhibitor of a tachykinin processing enzyme or a decomposition accelerator of a tachykinin processing enzyme. The neurokinin B signal blocker may be an expression inhibitor of neurokinin B (NKB), a neurokinin 3 receptor, a tachykinin processing enzyme, a gastrin-releasing peptide (GRP), or a GRP receptor. The neurokinin B signal blocker may be a removing agent for neurons expressing a neurokinin 3 receptor or a GRP receptor.

The neurokinin 3 receptor antagonist may be a compound represented by General Formula (1), (2), or (3) or a salt thereof, or a solvate thereof. The compound represented by General Formula (1) may be a compound represented by Formula (4), (5), (6), or (7), the compound represented by General Formula (2) may be a compound represented by Formula (8), (9), or (10), and the compound represented by General Formula (3) may be a compound represented by Formula (11).

The inhibitor may be an inhibitor of proprotein convertase subtilisin/kexin 1, proprotein convertase subtilisin/kexin 2, carboxypeptidase E, or peptidylglycine alpha-amidating monooxygenase.

The removing agent may be GRP, a specific binding substance to a GRP receptor, NKB, or a specific binding substance to a neurokinin 3 receptor, to which a cytotoxic substance is bound. The cytotoxic substance may be a ribosome-inactivating protein or a diphtheria toxin. The ribosome-inactivating protein may be saporin, ricin, or abrin.

### Examples

Hereinafter, the present invention will be described with reference to examples, but the present invention is not limited to the following examples.

### [Materials and Methods]

### (Mice)

Tac2^{-/-} mice were prepared by genome editing with a CRISPR/Cas9 system. A target site was selected within exon 4 of a mouse Tac2 gene using a CHOPCHOP web design tool (https://chopchop.rc.fas.harvard.edu/). Oligonucleotides shown in SEQ ID NO: 3 and SEQ ID NO: 4 and BbsI ligation adapter were synthesized. The sequences shown by SEQ ID NO: 5 and SEQ ID NO: 6 are guide sequences. In order to co-express a sgRNA and a Cas9 protein, two nucleotides thereof were synthesized, annealed, and then inserted into a BbsI-digested px330 vector. The prepared px330 vector (concentration of 5 ng/µL, Dalbecco's PBS) was injected into a pronucleus of a fertilized C57BL/6 mouse ovum that was fertilized in vitro in M2 medium (Sigma-Aldrich, Inc.). The injected germ cells were grown in CZB medium in an environment of 37 °C and 5% of CO₂ until embryos in the 2-cell stage matured. Subsequently, 24 to 36 embryos were transplanted into oviducts of female ICR mice. The genotypes of mouse pups were identified by PCR using the primers shown in Sequence 6 and Sequence 7, and by TA cloning and DNA sequencing. Mouse pups with desired mutations (Δ4 and Δ15) were mated with C57BL/6 mice or Dock8^{-/-} AND Tg mice.

Regarding the preparation of IL31^{-/-} mice, firstly, based on a pNT1.1 vector, an enhanced green fluorescent protein (EGFP) and a flippase regression target (FRT)-franked neomycin resistant cassette (neo) were inserted just behind an initiation codon to prepare a targeting vector. This targeting vector was introduced into embryonic stem cells (ES cells) by electroporation. The ES clone into which the vector was correctly introduced was injected into a blastocyst by microinjection, and the obtained male chimeric individuals were mated with female C57BL/6 mice. Mutant heterozygous mice were mated with CAG-FLPe transgenic mice (RBRC01834) to remove neo. Neo-removed mutant mice were backcrossed with C57BL/6 mice 5 times or more before mating with Dock8⁺¹⁻ AND Tg mice or Dock8^{+/-} Osmr^{+/-} mice. Osmr is a gene encoding an Oncostatin M receptor (OSMR), which forms a heterodimer with the IL-31 receptor α and transmits the IL-31 signal intracellularly.

All mice were bred in a special and pathogen-free environment within the animal facility of Kyushu University. As a control of the experiment, litters of the same age and gender were used. The methods of animal experiments were approved by the Animal Experiment Ethics Committee of Kyushu University.

### (Denervation of Nerves)

GRP-saporin, Nppb-saporin, and saporin only (2 µg/5 µL in each, Advanced Targeting Systems) were intrathecally administered to the L3/4 site to denerve spinal cord neurons that expressed a GRP receptor and a Nppb receptor. Two weeks after the toxic agent injection, the mice were used in experiments.

### (Measurement of Scratching Behavior)

Prior to the experiments, mice were placed in an acrylic cage (11 cm × 14 cm × 20 cm) for at least 1 hour to acclimatize to the environment. Subsequently, 50 µL of a pruritus-inducing substance dissolved in sterilized physiological saline was injected intradermally into shoulders of the mice, and the behavior of these mice was videotaped. The total number of scratching behavior within a specified time was determined by playing the video. During the scratching behavior of a mouse, the mouse extends its hind leg to the itchy area, tilts its head toward the hind leg, quickly turns the hind leg several times, and returns the hind leg to the ground. These series of actions were counted as one scratching behavior. As pruritus-inducing substances, IL-31 (1 µg/50 µl; PeproTech Inc.), SLIGRL-NH2 (100 µg/50 µl; Bachem Holding AG), chloroquine (100 µg/50 µl; Wako Pure Chemical Industries.), and histamine (100 µg/50 µl; Wako Pure Chemical Industries.) were used.

In some experiments, the actions of NK3R antagonists on the scratching behavior of the mice were investigated. As compounds having activity as an NK3R antagonist or an NK3R antagonist, osanetant (Axon Medchem.), talnetant (NAMIKI SHOJI Co., Ltd.), pavinetant (NAMIKI SHOJI Co., Ltd.), fezolinetant (Haoyuan Chemexpress Co., Ltd.), SSR-146977(Tocris Bioscience.), SSR-241586 (WuXi AppTec New Drug Development Co. Ltd.), SB-235375 (WuXi AppTec New Drug Development Co. Ltd.), and CS-003 (WuXi AppTec New Drug Development Co. Ltd.) were used. These compounds were administered to the mice in a predetermined amount per weight of each mouse 45 minutes before the intradermal injection of the pruritus-inducing substance. Osanetant was dissolved in phosphate buffered saline (PBS) containing 0.1% Tween-20 and intraperitoneally injected at 5 mg/kg. Talnetant and pavinetant were dissolved in a mixture of 10% dimethyl sulfoxide (DMSO), 40% polyethylene glycol 300, 5% Tween-80, and 45% saline and were intraperitoneally injected at 30 mg/kg. Fezolinetant was dissolved in PBS containing 10% DMSO and orally administered at 10 mg/kg. SSR-146977 was dissolved in PBS containing 0.1% ethanol and intraperitoneally injected at 0.3 mg/kg. SSR-241586 was dissolved in a 0.01% Tween-80 aqueous solution and intraperitoneally injected at 3 mg/kg. SB-235375 was dissolved in PBS and orally administered at 3 mg/kg. CS-003 was dissolved in PBS containing 5% glucose and intravenously injected at 3 mg/kg.

### (Histology and Immunohistochemistry)

Skin tissues were fixed with 4% (w/v) paraformaldehyde and embedded in a paraffin block. Sections with a thickness of 3 µm were stained with hematoxylin and eosin, and observed with an optical microscope. In immunohistochemical analysis of dorsal root ganglia (DRG) and spinal cords, mice were anesthetized with isoflurane, and PBS and 4% paraformaldehyde were sequentially refluxed from the heart. The DRG and spinal cord tissues were collected, fixed overnight at 4 °C, and then immersed in 30% sucrose-PBS to prevent tissue frost damage. Tissue samples were embedded with an OCT compound (Sakura Finetech Japan Co., Ltd.) and frozen on dry ice. Sections with a thickness of 10 µm were prepared using a cryostat, the sections were blocked by G-block (Genostaff, GB-01) for 30 minutes at room temperature, and incubated overnight at 4 °C with primary antibodies. The primary antibodies were detected with secondary antibodies (Thermo Fisher Scientific K.K.) conjugated with a fluorescent dye. DAPI (1:5000, DOJINDO LABORATORIES) was used for each staining. All images were acquired using a confocal laser scanning microscope (FV3000, Olympus Group). As the primary antibodies, rabbit anti-neurokinin B antibodies (1:500; Novus Biologicals, NB300-201), rabbit anti-NK3R antibodies (1:50; Novus Biologicals, NB300-102), rabbit anti-TRPVl antibodies (1:500; Millipore Corporation, AB9554), anti-IL-31RA antibodies (1:100; R & D Systems, AF2107), mouse anti-c-fos antibodies (1:1000; EnCor Biotechnology Inc., MCA-2H2), rabbit anti-GRPR antibodies (1:50; LifeSpan BioSciences, Inc., LS-A831-50), and rabbit anti-NPRA antibodies (1:50; LifeSpan BioSciences, Inc., LS-C164506) were used.

### (Microarray Analysis)

Total RNAs were separated using ISOGEN (NIPPON GENE CO., LTD.), and cRNA was amplified and labeled using Low Input Quick Amp Labeling Kit (Agilent Technologies, Inc.). The cRNA was hybridized to 44K 60-mer oligomicroarray (Whole Mouse Genome oligo DNA Microarray Kit Ver 2.0; Agilent Technologies, Inc.). Hybridized microarray slides were scanned using Agilent scanner. A relative intensity of hybridization and a background value of hybridization were calculated using Feature Extraction Software version 9.5.1.1 (Agilent Technologies, Inc.). Raw signal intensity data and flag values of probes were calculated from the hybridization intensity and a spot location according to the approach recommended by Agilent Technologies, Inc. In order to identify genes whose expression levels were increased or decreased in the test samples, Z-scores and ratios were calculated based on the normalized signal intensity of each probe. Genes with a Z-score greater than 2.0 and a ratio greater than 1.5 were considered to have an increased expression level, and genes with a Z-score smaller than - 2.0 and a ratio smaller than 0.66 were considered to have a decreased expression level.

### (Statistical Analysis)

Statistical analysis was performed using GraphPad Prism. First, a Kolmogorov-Smirnov test was used to examine whether the data followed a normal distribution. Parametric data were analyzed using a two-tailed unpaired Student's t-test in a case of comparing data from two populations. Parametric data were analyzed using one-way ANOVA and then a Bonferroni post hoc test in a case of comparing data from a plurality of populations. Nonparametric data were analyzed using a Mann-Whitney test in a case of comparing data from two populations. A P-value of 0.05 or smaller was considered significant.

### (Real-Time PCR)

Total RNAs were extracted from each tissue using ISOGEN (NIPPON GENE CO., LTD.). Total RNAs were treated with RNase-free DNase I (Thermo Fisher Scientific K.K.). This RNA sample was reverse transcribed using an oligo (dT) primer (v) and SuperScript III reverse transcriptase (Thermo Fisher Scientific K.K.), and amplified by PCR. Real-time PCR was performed by Cfx Connecttm Thermal Cycler (Bio-Rad Laboratories, Inc.) using SYBR Green PCR Master Mix (Applied Biosystems). The expression level of the target gene was normalized by an expression level of Hprt gene. For the analysis, CFX Manager (ver3.1) attached to a device was used.

### [Experimental Example 1]

### (Itching Transmitting Neural Circuit)

A neural circuit that transmits a pruritus sensation that is activated by IL-31 was analyzed. Many itching-inducing substances transmit a pruritus sensation to spinal nerves through natriuretic polypeptide b (Nppb) or gastrin-releasing peptide (GRP).

In order to remove neurons expressing GRP receptors or Nppb receptors, each of saporin-conjugated GRP or Nppb (GRP-saporin or Nppb-saporin) was injected to medullary cavities of wild-type C57BL/6 mice. Two weeks later, IL-31 was administered to these mice, and the scratching behavior of each mouse was analyzed.

Saporin is a ribosome-inactivating protein (RIP).

Proteins conjugated with ligands of receptors expressed on target neurons and RIPs are captured by the target neurons and incorporated into cells by endocytosis. The RIP-conjugated proteins that are incorporated into cells inhibit translation and kill the target neurons.

Fig. 1(a) shows a result obtained from injecting each of GRP-saporin or Nppb-saporin into medullary cavities of wild-type C57BL/6 mice, administering IL-31 to these mice, and then analyzing scratching behavior of these mice. In Fig. 1(a), Blank shows a result of injecting only saporin into the medullary cavity. IL-31 (-) shows a result of an IL-31 non-administration group, and IL-31 (+) shows a result of an IL-31 administration group. In Figs. 1(a) to (c), "*" indicates that there is a significant difference at p < 0.05, and "**" indicates that there is a significant difference at p < 0.01.

From the result of the intramedullary injection experiment, it was clarified that the scratching behavior induced by IL-31 was suppressed due to the injection of GRP-saporin. However, in a case where Nppb-saporin was injected, the scratching behavior was not suppressed.

Next, an expression level of GRP receptor (GRPR) mRNA or NPP receptor A (NPRA) mRNA and an expression level of the protein were analyzed to confirm whether or not GRP-saporin and Nppb-saporin removed nerves expressing GRPR and NPRA, respectively.

The expression levels of GRPR gene mRNA and NPRA gene mRNA were analyzed as follows. First, spinal cords were excised from the mice after the injection, total RNAs were extracted from the excised spinal cords, and a reverse transcription reaction was performed using the total RNAs as a template. Subsequently, quantitative PCR was performed to analyze the expression level of each gene.

Fig. 1(b) and (c) show a result obtained from injecting GRP-saporin or Nppb-saporin into the medullary cavities of wild-type C57BL/6 mice and analyzing expression levels of GRPR mRNA and NPRA mRNA in a spinal cord.

From the result of analyzing the expression levels of GRPR mRNA and NPRA mRNA, it was confirmed that the expression levels of GRPR mRNA and NPRA mRNA in the spinal cord were decreased by the injection of GRP-saporin or Nppb-saporin.

The expression levels of a GRPR protein and an NPRA protein were analyzed as follows. The spinal cords after the injection were excised from the mice, and sections were prepared from the excised spinal cords. The prepared sections were analyzed by immunohistochemistry using anti-GRPR antibodies and anti-NPRA antibodies.

Fig. 1(d) shows a result obtained from injecting GRP-saporin or Nppb-saporin into the medullary cavity, excising the spinal cord, and subjecting it to immunostaining with anti-GRPR antibodies and anti-NPRA antibodies. In Fig. 1(d), the scale bar indicates 10 µm.

From the result of analyzing the expression levels of the GRPR protein and the NPRA protein, it was confirmed that the expression levels of the GRPR protein and the NPRA protein in the spinal cord were decreased by the injection of GRP-saporin or Nppb-saporin.

Summarizing the above results, it was confirmed that neurons expressing GRPR and NPRA were denervated by the injection of GRP-saporin or Nppb-saporin. In addition, it was clarified that the scratching behavior induced by the administration of IL-31 is not mediated by Nppb, but is mediated by GRP.

### [Experimental Example 2]

### (Preparation of IL-31 mutant mice)

In order to analyze the function of 1L-31, mice lacking the IL-31 gene were prepared. As described above, a neo cassette sandwiched between FRTs was inserted immediately after exon 1 of the IL-31 gene. Fig. 2(a) is a figure schematically showing the IL-31 gene, a targeting vector, and a gene structure after recombination.

In Fig. 2(a), WT allele is a schematic figure of a gene structure of IL-31 in wild-type C57BL/6 mice, Targeting vector is a schematic diagram figure (or result) of a structure of the targeting vector, Recombinant allele is a schematic diagram figure (or result) of a gene structure after homologous recombination, and KI allele is a schematic diagram figure(or result) of a gene structure after the neo cassette is removed by flippase.

The genotypes of wild-type C57BL/6 mice and neo-removed mice were determined by PCR using primers shown in SEQ ID NOs: 9, 10, and 11. Fig. 2(b) is a figure showing that each PCR product has been electrophoresed on agarose gel of 2%. In Fig. 2(b), w/w indicates the genotype of wild-type allele, KI/w indicates the genotype of heterozygosity between wild-type allele and KI allele, and KI/KI indicates the genotype of KI allele homozygosity.

The length of the PCR product using a wild-type C57BL/6 mouse-derived genome as a template was 300 bp, and the length of the PCR product using a knock-in mouse-derived genome from which IL-31 gene was removed as a template was 550 base pairs. From this result, it was confirmed that the knock-in mice were obtained.

### [Experimental Example 3]

### (Analysis of Dock8^{-/-} AND Tg Mice)

Dermatitis and scratching behavior of Dock8^{-/-} AND Tg mice were analyzed. In addition, genes whose expression levels were increased in the dorsal root ganglia of Dock8^{-/-} AND Tg mice were analyzed.

First, the skin tissues of AND Tg mice having the genotypes of Dock8^{+/-} Osmr^{+/+} IL31^{+/+}, Dock8^{-/-} Osmr^{+/-} 1L31^{+/+}, Dock8^{-/-} Osmr^{-/-} IL31^{+/+}, and Dock8^{-/-} Osmr^{+/+} IL31^{-/-} were analyzed by HE staining. Fig. 3(a) is a figure showing analysis of the skin tissues of the mice of each genotype by hematoxylin staining. Osmr is a gene encoding an Oncostatin M receptor (OSMR), which forms a heterodimer with the IL-31 receptor α and transmits the IL-31 signal intracellularly.

From the result of analyzing the skin tissues, it was clarified that atopic dermatitis-like dermatitis was induced in AND Tg mice lacking the Dock8 gene, whereas this dermatitis disappeared due to deficiency of the Osmr or IL31 gene.

Next, the scratching behavior of AND Tg mice having the genotypes of Dock8^{+/-} Osmr^{+/+} IL31^{+/+}, Dock8^{-/-} Osmr^{+/-} IL31^{+/+}, Dock8^{-/-} Osmr^{-/-} IL31^{+/+}, and Dock8^{-/-} Osmr^{+/+} IL31^{-/-} was analyzed. Fig. 3(b) is a figure showing analysis of the scratching behavior of mice in each genotype.

From the result of analyzing the scratching behavior, it was clarified that AND Tg mice lacking the Dock8 gene exhibited the scratching behavior, whereas this scratching behavior disappeared due to deficiency of the Osmr or IL31 gene.

Furthermore, a concentration of serum IL-31 AND Tg mice having the genotypes of Dock8^{+/-} Osmr^{+/+} IL31^{+/+}, Dock8^{-/-} Osmr^{+/-} IL31^{+/+}, Dock8^{-/-} Osmr^{-/-} IL31^{+/+}, and Dock8^{-/-} Osmr^{+/+} IL31^{-/-} was analyzed. Fig. 3(c) shows a result obtained from analyzing a concentration of serum IL-31 of mice in each genotype.

From the result of analyzing the concentration of IL-31, the concentration of serum IL-31 of AND Tg mice lacking the Dock8 gene increased, whereas this increase in the concentration disappeared due to deficiency of the IL-31 gene.

Next, a gene whose expression level in the dorsal root ganglia (DRG) of Dock8^{-/-} AND Tg mice is higher than the expression level in DRG of Dock8^{+/-} AND Tg mice was analyzed using a microarray.

From the analysis results of the microarray, it was clarified that expression levels of known signal transduction factor mRNA and neurotransmitter mRNA were increased. One of them was Tac2 encoding neurokinin B.

Next, an expression level of Tac2 mRNA in the DRG of AND Tg mice having the-genotype of Dock8^{+/-} Osmr^{+/+} 1L31^{+/+}, Dock8^{-/-} Osmr^{+/-} 1L31^{+/+}, Dock8^{-/-} Osmr^{-/-} 1L31^{+/+}, and Dock8^{-/-} Osmr^{+/+} IL31^{-/-} was analyzed. Fig. 3(d) shows a result obtained from analyzing the expression level of Tac2 mRNA in the DRG of mice in each genotype. In Figs. 3(b) to (d), "*" indicates that there is a significant difference at p < 0.05, and "**" indicates that there is a significant difference at p < 0.01, as compared with data of Dock8^{+/-} Osmr^{+/+} IL31^{+/+}.

The expression level of Tac2 mRNA was analyzed as follows. First, total RNAs were extracted from the DRG of mice in each genotype, and a reverse transcription reaction was performed using the total RNAs as a template. Subsequently, quantitative PCR was performed to analyze the expression level of Tac2 gene mRNA.

As a result, it was clarified that the expression level of Tac2 mRNA increases by a factor of about 23 in the DRG of AND Tg mice lacking the Dock8 gene. In addition, it was clarified that this increase in the expression level disappeared due to deficiency of the Osmr or IL31 gene.

Summarizing the above results, in Dock8^{-/-} AND Tg mice, it was clarified that since the production amount of an IL-31 protein was increased, the IL-31 protein increased the expression level of the Tac2 gene through an IL-31 receptor protein including OSMR.

### [Experimental Example 4]

### (Changes in Gene Expression by stimulation with IL-31 and NKB)

Changes in the expression levels of Tac2, Grp, and Nppb through administration of IL-31 or NKB were analyzed. First, DRG neurons isolated from the wild-type C57BL/6 mice were stimulated by the addition of the IL-31 protein in vitro, and the expression levels of Tac2 mRNA, Grp mRNA, and Nppb mRNA were analyzed. Fig. 4(a) shows a result obtained from analyzing the expression level of mRNA in each gene after the addition of IL-31.

From the result of analyzing the expression levels of Tac2 mRNA, Grp mRNA, and Nppb mRNA, it was clarified that the expression levels of Tac2 mRNA and Grp mRNA were increased by the addition of IL-31, whereas the expression level in Nppb was not increased.

Next, DRG neurons isolated from the wild-type C57BL/6 mice were stimulated by neurokinin B (NKB) in vitro, and the expression levels of Grp mRNA and Nppb mRNA were analyzed. Fig. 4(b) shows a result obtained from analyzing the expression level in each gene after the addition of NKB.

From the result of analyzing the expression levels of Grp mRNA and Nppb mRNA, it was clarified that the expression levels of Grp mRNA and Nppb mRNA were increased by the stimulation of NKB. From this result, it is considered that NKB functions upstream of GRP.

### [Experimental Example 5]

### (Expression of NKB in DRG of Dock8^{-/-} AND Tg Mice)

The expression of NKB and IL-31 receptor A (IL-31RA) in the DRG of Dock8^{-/-} AND Tg mice was analyzed by immunostaining.

The DRG was isolated from the Dock8^{-/-} AND Tg mice to prepare for microscope sections. The prepared sections were analyzed by immunohistochemistry using anti-NKB antibodies and anti-IL-31RA antibodies. Fig. 5(a) shows the result of immunostaining with anti-NKB antibodies and anti-IL-31RA antibodies.

In Fig. 5(a), "DAPI" indicates the result of DAPI staining, "NKB" indicates the result of immunostaining with anti-NKB antibodies, "IL-31RA" indicates the result of immunostaining with anti-IL-31RA antibodies, and "Merge" indicates the result of superimposing immunostaining with anti-NKB antibodies, immunostaining with anti-IL-31RA antibodies, and DAPI staining. The scale bar indicates 100 µm.

From the result of immunostaining, it was clarified that NKB is expressed in cells expressing the IL-31RA protein in the DRG of Dock8^{-/-} AND Tg mice.

Next, the expressions of a NKB protein and a Transient recipient potential cation Channel vanilloid subtype 1 (TRPV1) protein in the DRG of Dock8^{-/-} AND Tg mice were analyzed by immunostaining.

TRPV1 is expressed in sensory neurons that sense itching, and the sensory neurons extend axons to outer layer lamina I of a spinal cord dorsal horn.

The expressions of NKB and TRPV1 were analyzed as follows. The DRG was isolated from the Dock8^{-/-} AND Tg mice to prepare sections. The prepared sections were analyzed by immunohistochemistry using anti-NKB antibodies and anti-TRPV1 antibodies. Fig. 5(b) is a figure showing the expressions of NKB and TRPV1 in the DRG of Dock8^{-/-} AND Tg mice.

In Fig. 5(b), "DAPI" indicates the result of DAPI staining, "NKB" indicates the result of immunostaining with anti-NKB antibodies, "TRPV1" indicates the result of immunostaining with anti-TRPV1 antibodies, and "Merge" indicates the result of superimposing immunostaining with anti-NKB antibodies and immunostaining with anti-TRPV1 antibodies.

From the result of immunostaining, it was clarified that NKB is expressed in some of the cells expressing TRPV1 in the DRG of Dock8^{-/-} AND Tg mice.

### [Experimental Example 6]

### (Preparation of Tac2 Gene Mutant Mice)

In order to analyze a function of a Tac2 gene, CRISPR/Cas9 nuclease was used to prepare mice lacking the Tac2 gene (Tac2^{-/-}).

Fig. 6(a) is a figure schematically showing the mouse Tac2 gene and the protein encoded by the mouse Tac2 gene. The mature and active Tac2 protein is encoded by exon 6, which is synthesized from the precursor peptide through processing. A guide RNA used for genome editing by the CRISPR/Cas9 system was designed inside exon 4.

Two strains of mutant mice (Δ4 and Δ15) were prepared by the above described genome editing. In PCR, primers with the sequences shown in SEQ ID NO: 7 and SEQ ID NO: 8 were used. Electrophoresis was performed using a MultiNA microchip electrophoresis system (MCE-202, Shimadzu Corporation). Fig. 6(b) is a figure showing identification of the Tac2 genotype of Tac2 gene-deficient mice Δ4 and Δ15 by PCR and electrophoresis.

Fig. 6(c) is a figure showing genomic sequences of two mutations and amino acid sequences encoded by the transcribed mRNA. Deletion sites are indicated by Δ4 (4 base deficiency) and Δ15 (15 base deficiency). In Fig. 6(c), coding sequences are shown by solid lines and splice donor sequences are shown by boxes.

The sequences of the transcribed mRNAs were determined as follows. First, total RNAs were extracted from the DRG neurons in each mutant mouse, and cDNAs were obtained by a reverse transcription reaction. These cDNA were amplified by PCR using the primers shown in SEQ ID NO: 12 and SEQ ID NO: 13. The amplification products were cloned by TA cloning and cDNA sequences were analyzed.

Both mutations have deletion mutations in exon 4 and cause a frameshift in the amino acid sequence. Therefore, it was clarified that the mutated genes do not encode the mature peptide sequences.

### [Experimental Example 7]

### (Analysis of Tac2 Mutant Mice)

Tac2 mutant mice (Δ4 and Δ15) were used to analyze a role of the NKB protein encoded by the Tac2 gene in the transmission of itching sensation induced by the administration of IL-31.

Histamine, chloroquine, SLIGRL-NH₂ (PAR2 agonist), or IL-31, which is the pruritus-inducing substance, was intradermally injected into Tac2^{-/-} (Δ4) mice and Tac2^{+/-} mice. The results are shown in Fig. 7.

Fig. 7(a) shows a result obtained from analyzing the scratching behavior after the administration of histamine to Tac2^{-/-} (Δ4) mice. Fig. 7(b) shows a result obtained from analyzing the scratching behavior after the administration of chloroquine to Tac2^{-/-} (Δ4) mice. Fig. 7(c) shows a result obtained from analyzing the scratching behavior after administration of PAR2 agonist (SLIGRL-NH2) to Tac2^{-/-} (Δ4) mice. Fig. 7(d) shows a result obtained from analyzing the scratching behavior after administration of IL-31 to Tac2^{-/-} (Δ4) mice. In Figs. 7(a) to (d), "*" indicates that there is a significant difference at p < 0.05.

Administration of histamine, chloroquine, and PAR2 agonist (SLIGRL-NH₂) induced the scratching behavior in Tac2^{-/-} (Δ4) mice and Tac2^{+/-} mice, as previously reported. It was clarified that the administration of IL-31 induced the scratching behavior in Tac2^{+/-} mice, whereas it hardly induced the scratching behavior in Tac2^{-/-} (Δ4) mice.

The same experiment as described above was performed on Tac2^{-/-} (Δ15) mice and Tac2^{+/-} mice. The result of analyzing the scratching behavior is shown in Fig. 8. Fig. 8(a) shows a result obtained from analyzing the scratching behavior after the administration of histamine to Tac2^{-/-} (Δ15) mice. Fig. 8(b) shows a result obtained from analyzing the scratching behavior after administration of IL-31 to Tac2^{-/-} (Δ15) mice.

As in the case of Tac2^{-/-} (Δ4) mice, it was clarified that the administration of histamine induced the scratching behavior in Tac2^{-/-} (Δ15) mice, whereas the administration of IL-31 hardly induced the scratching behavior.

The Dock8^{-/-} AND Tg mice that have expressed Tac2 or have not expressed Tac2 (Tac2^{-/-} (Δ4)) were established by the Tac2^{-/-} (Δ4) mice being mated with the Dock8^{-/-} AND Tg mice, and the skin tissues, the scratching behavior, and the serum IL-31 levels were analyzed. The results are shown in Fig. 9.

Fig. 9 shows a result of comparing Dock8^{-/-} AND Tg mice that have expressed Tac2 or have not expressed Tac2 to each other for the skin tissues in Fig. 9(a), the scratching behavior in Fig. 9(b), and the serum IL-31 levels in Fig. 9(c). In Figs. 9(a) to (c), "*" indicates that there is a significant difference at p < 0.05. It can be seen that, in the absence of Tac2, the level of IL-31 does not change, whereas dermatitis and scratching behavior are significantly improved.

### [Experimental Example 8]

### (Mechanism of Action of Neurokinin B)

Next, in order to identify the site where NKB was functioning, NKB was injected into medullary cavities of Tac2^{-/-} (Δ4) mice, and scratching behavior was analyzed. Fig. 10(a) shows a result obtained from injecting NKB into medullary cavities of Tac2^{-/-} (Δ4) mice and analyzing scratching behavior.

It was clarified that the intramedullary injection of NKB protein into Tac2^{-/-} (Δ4) mice induced the scratching behavior. This result indicates that NKB induces the scratching behavior without activating peripheral nerves.

Next, the neural circuit that induces the scratching behavior by the NKB administration was analyzed. GRP-saporin or Nppb-saporin was injected into the medullary cavities of wild-type C57BL/6 mice, and two weeks later, NKB was injected, and scratching behavior was analyzed.

Fig. 10(b) shows a result obtained from injecting GRP-saporin or Nppb-saporin, injecting NKB, and then analyzing scratching behavior. In Figs. 10(a) and (b), "*" indicates that there is a significant difference at p < 0.05, and "**" indicates that there is a significant difference at p < 0.01.

Mice from which neurons expressing the GRP receptor were removed did not show the scratching behavior after the administration of NKB, whereas mice from which neurons expressing the Nppb receptor were removed showed the scratching behavior after the administration of NKB. From this result, it was clarified that NKB is a neurotransmitter that functions upstream of GRP and transmits itching sensation induced by IL-31.

### [Experimental Example 9]

### (Analysis of NK3R)

The function and localization of NK3R were analyzed.

The expression of NK3R and TRPV1 was analyzed as follows. The spinal cord dorsal horns were excised from the mice, and sections were prepared from the excised spinal cord dorsal horns. The prepared sections were analyzed by immunohistochemistry using anti-NK3R antibodies and anti-TRPVl antibodies. Fig. 11(a) is a result of analyzing the expressions of NK3R and TRPV1 in the spinal cord dorsal horn. In Fig. 11(a), the scale bar indicates 100 µm.

It was clarified that NK3R was mainly expressed in the neurons in the outer layer lamina I of the spinal cord dorsal horn.

Next, it was analyzed whether or not itching sensation induced by IL-31 was mediated by the Neurokinin 3 receptor (NK3R).

After the administration of IL-31, c-fos activation in NK3R-expressing neurons in the spinal cord dorsal horn was analyzed. The result is shown in Fig. 11(b).

In Fig. 11 (b), "DAPI" is a figure of staining performed with DAPI, "NK3R" is a figure of immunostaining performed with anti-NK3R antibodies, "c-fos" is a diagram figure (or result) of immunostaining performed with anti-c-fos antibodies, and "Merge" is a diagram figure (or result) in which staining with DAPI, immunostaining with anti-NK3R antibodies, and immunostaining with anti-c-fos antibodies are superimposed. As a result, it was clarified that c-fos was activated by the administration of IL-31 in neurons expressing NK3R. The scale bar indicates 10 µm.

Next, osanetant, a selective antagonist of NK3R and an anti-psychotic drug, was intraperitoneally injected into wild-type C57BL/6 mice, and then the effect of the compound on the scratching behavior induced by IL-31, histamine, or chloroquine was analyzed.

Fig. 11(c) shows a result obtained from injecting osanetant, administering IL-31, and then analyzing scratching behavior. Fig. 11(d) shows a result obtained from injecting osanetant, administering histamine, and then analyzing scratching behavior. Fig. 11(e) shows a result obtained from injecting osanetant, administering chloroquine, and then analyzing scratching behavior. In Fig. 11(c), (d), and (e), "*" indicates that there is a significant difference at p < 0.05, and "**" indicates that there is a significant difference at p < 0.01.

As a result, it was clarified that the administration of osanetant decreased the frequency of scratching behavior by IL-31, whereas it did not decrease the frequency of scratching behavior by histamine and chloroquine.

Similar to osanetant, fezolinetant that is a selective antagonist of NK3R was orally administered to wild-type C57BL/6 mice, and the effect on the scratching behavior induced by IL-31, histamine, or chloroquine was analyzed.

Fig. 12(a) shows a result obtained from orally administering fezolinetant, administering IL-31, and then analyzing scratching behavior. Fig. 12(b) shows a result obtained from administering fezolinetant, administering histamine, and then analyzing scratching behavior. Fig. 12(c) shows a result obtained from administering fezolinetant, administering chloroquine, and then analyzing scratching behavior.

As a result, it was clarified that the administration of fezolinetant decreased the frequency of scratching behavior by IL-31, whereas it did not decrease the frequency of scratching behavior by histamine and chloroquine, which is the same result as the case of the administration of osanetant.

Furthermore, talnetant, pavinetant, SSR-146977, and SB-235375 that are other NK3R selective antagonists were administered to wild-type C57BL/6 mice, and then the effects of the compounds on the scratching behavior induced by IL-31 or chloroquine were analyzed.

Fig. 13(a) shows a result obtained from injecting talnetant, administering IL-31, and scratching behavior is analyzed. Fig. 13(b) shows a result obtained from injecting talnetant, administering chloroquine, and then analyzing scratching behavior. Fig. 14(a) shows a result obtained from injecting pavinetant, administering IL-31, and then analyzing scratching behavior. Fig. 14(b) shows a result obtained from injecting pavinetant, administering chloroquine, and then analyzing scratching behavior. Fig. 15(a) shows a result obtained from injecting SSR-146977, administering IL-31, and then analyzing scratching behavior. Fig. 15(b) shows a result obtained from injecting SSR-146977, administering chloroquine, and then analyzing scratching behavior is analyzed. Fig. 18(a) shows a result obtained from orally administering SB-235375, administering IL-31, and then analyzing scratching behavior. Fig. 18(b) shows a result obtained from orally administering SB-235375, administering chloroquine, and analyzing scratching behavior.

From the results shown in Figs. 13, 14, 15, and 18, it was clarified that the administration of talnetant, pavinetant, SSR-146977, and SB-235375, which are the NK3R-selective antagonists, did not decrease the frequency of scratching behavior by chloroquine, whereas it did decrease the frequency of scratching behavior induced by IL-31. These results were identical to the results obtained in the case where osanetant and fezolinetant were administered.

Furthermore, CS-003 that is an antagonist to neurokinin 3 receptor (NK3R) and also an antagonist to neurokinin 1 receptor (NK1R) and neurokinin 2 receptor (NK2R), and SSR-241586 that is an antagonist to NK3R and NK2R were administered to wild-type C57BL/6 mice, and then the effects of the compounds on the scratching behavior induced by IL-31 or chloroquine were analyzed.

Fig. 16(a) shows a result obtained from injecting SSR-241586, administering IL-31, and then analyzing scratching behavior is analyzed. Fig. 16(b) shows a result obtained from injecting SSR-241586, administering chloroquine, and then analyzing scratching behavior. Fig. 17(a) shows a result obtained from injecting CS-003, administering IL-31, and then analyzing scratching behavior. Fig. 16(b) shows a result obtained from injecting CS-003, administering chloroquine, and then analyzing scratching behavior.

From the results shown in Figs. 16 and 17, it was clarified that even in an NK3R-non-selective antagonist, the compound having activity as an NK3R antagonist did not decrease the frequency of scratching behavior by chloroquine, whereas it did decrease the frequency of scratching behavior induced by IL-31.

In each of Figs. 12 to 18, "*" indicates that there is a significant difference at p < 0.05, and "**" indicates that there is a significant difference at p < 0.01.

In summary, it was clarified that the administration of IL-31 activates neurons expressing NK3R. In addition, it was clarified that the pharmacological inhibition of NK3R selectively suppresses the scratching behavior by IL-31.

### [Industrial Applicability]

According to the present invention, it is possible to provide the prophylactic or therapeutic agent for an IL-31-mediated disease including a neurokinin B signal blocker as an active ingredient.

## Claims

1. A prophylactic or therapeutic agent for an IL-31-mediated disease comprising a neurokinin B signal blocker.

2. The prophylactic or therapeutic agent according to Claim 1, wherein the IL-31-mediated disease includes atopic dermatitis, pruritus in atopic dermatitis, dermatomyositis, pruritus in dermatomyositis, chronic dermatitis, allergic contact dermatitis, dermatitis herpetiformis, pruritus in cutaneous T cell lymphoma, prurigo nodularis, prurigo chronica multiformis, urticaria pigmentosa, or bullous pemphigoid.

3. The prophylactic or therapeutic agent according to Claim 1, wherein the IL-31-mediated disease is atopic dermatitis.

4. The prophylactic or therapeutic agent according to any one of Claims 1 to 3, wherein the neurokinin B signal blocker is a neurokinin 3 receptor antagonist.

5. The prophylactic or therapeutic agent according to Claim 4, wherein the antagonist is a compound represented by General Formula (1), (2), or (3) or a salt thereof, or a solvate of thereof, 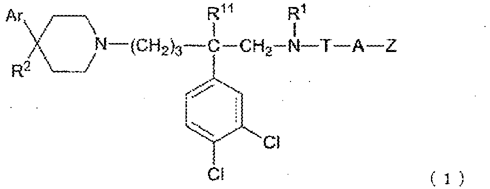 [in Formula (1),
Ar represents a piperidinyl group, a pyridin-2-yl group, a phenyl group, or a phenyl group substituted with a halogen atom, a methyl group, or an alkoxy group having 1 to 4 carbon atoms;
R¹ represents a methyl group and R¹¹ represents a hydrogen atom, or R¹ and R¹¹ together represent a -(CH₂)₃- group or a -(CH₂)₂O- group;
R² represents a hydroxyl group, a C₁₋₇ alkoxy group, a C₁₋₇ acyloxy group, a cyano group, a -NR⁶R⁴ group, a -NR³COR⁴ group, a -NR³COOR⁸ group, a -NR³SO₂R⁹ group, a -NR³CONR¹⁰R¹² group, a C₁₋₇ acyl group, a C₁₋₇ alkoxycarbonyl group, a - CONR¹⁰R¹² group, a CH₂OH group, a C₁₋₇ alkoxymethyl group, a C₁₋₇ acyloxymethyl group, a C₁₋₇ alkylaminocarbonyloxymethyl group, a -CH₂NR¹³R¹⁴ group, a - CH₂NR³COR⁴ group, a -CH₂NR³COOR⁸ group, a -CH₂NR³SO₂R⁹ group, or a - CH₂NR³CONR¹⁰R¹² group, R² constitutes a double bond between the carbon atom to which it is attached and the adjacent carbon atom of the piperidine ring, or Ar and R² together with a piperidine ring to which Ar and R² are bonded form a group represented by Formula (1a) or (1b), 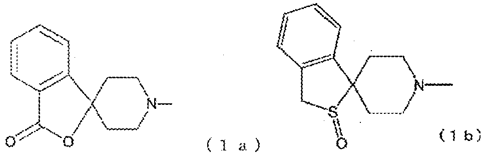
where R³ represents a hydrogen atom or a C₁₋₄ alkyl group and R⁴ represents a hydrogen atom, a C₁₋₇ alkyl group, a phenyl group, a benzyl group, a pyridyl group, an unsubstituted C₃₋₇ cycloalkyl group, or a C₃₋₇ cycloalkyl group substituted with one or more methyl groups, or R³ and R⁴ together represent a -(CH₂)ₙ- group (where n is 3 or 4);
T represents a methylene group, a carbonyl group, a -COO- group, or a - CONR⁵- group and A represents a single bond, a methylene group, an ethylene group, a propylene group, or a vinylene group, or -T-A- represents a -SO₂- group; and
Z represents a phenyl group or a phenyl group substituted with one or more of a halogen atom, a C₁₋₄ alkyl group, a C₁₋₄ alkoxy group, and a nitro group,
where R⁵ represents a hydrogen atom or a C₁₋₄ alkyl group,
R⁶ represents a hydrogen atom or a C₁₋₇ alkyl group and R⁷ represents a hydrogen atom, a C₁₋₇ alkyl group, a C₃₋₇ cycloalkylmethyl group, a benzyl group, or a phenyl group, or R⁶ and R⁷ together with a nitrogen atom to which R⁶ and R⁷ are bonded form a heterocycle selected from the group consisting of an azetidine group, a pyrrolidine group, a piperidine group, a morpholine group, a thiomorpholine group, and a perhydroazepine group,
R⁸ represents a C₁₋₇ alkyl group or a phenyl group,
R⁹ represents a C₁₋₇ alkyl group, an amino group or an amino group substituted with one or two C₁₋₇ alkyl groups, a phenyl group or a phenyl group substituted with one or more groups selected from the group consisting of a halogen atom, a C₁₋₇ alkyl group, a trifluoromethyl group, a hydroxyl group, a C₁₋₇ alkoxy group, a carboxyl group, a C₁₋₇ alkoxycarbonyl group, a C₁₋₇ alkylcarbonyloxy group, a cyano group, a nitro group, an amino group, and an amino group substituted with one or two C₁₋₇ alkyl groups (where, in a case where a plurality of groups are selected, the plurality of groups are the same as or different from each other),
R¹⁰ represents a hydrogen atom or a C₁₋₇ alkyl group and R¹² represents a hydrogen atom, a C₁₋₇ alkyl group, a C₃₋₇ cycloalkyl group, a C₃₋₇ cycloalkylmethyl group, a hydroxyl group, a C₁₋₄ alkoxy group, a benzyl group, or a phenyl group, or R¹⁰ and R¹² together with a nitrogen atom to which R¹⁰ and R¹² are bonded form a heterocycle selected from the group consisting of an azetidine group, a pyrrolidine group, a piperidine group, a morpholine group, a thiomorpholine group, and a perhydroazepine group,
R¹³ represents a hydrogen atom or a C₁₋₇ alkyl group,
R¹⁴ represents a hydrogen atom, a C₁₋₇ alkyl group, a C₃₋₇ cycloalkylmethyl group, or a benzyl group,
n3 is 2 or 3, and
in a case where Ar is a phenyl group, R² is a hydroxyl group, and -T-A-Z is a benzoyl group, R¹ is not a methyl group; in a case where Ar is a phenyl group, R² is a - NHCOCH3 group, and -T-A-Z is a benzoyl group, R¹ and R¹¹ together do not form a - (CH₂)₃- group; in a case where Ar is a phenyl group, R² is a hydroxyl group, and -T-A-Z is a 3-methoxybenzyl group, R¹ and R¹¹ together do not form a -(CH₂)₃- group; and in a case where Ar is a phenyl group, R² is a -NHCOCH3 group, and -T-A-Z is a benzyloxycarbonyl group, R¹ is not a methyl group], 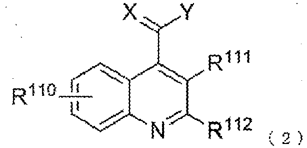
[in Formula (2),
Y is a group represented by Formula (2a) or (2b), 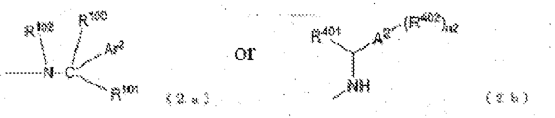
where Ar² represents a phenyl group, a naphthyl group, or a C₅₋₇ cycloalkadienyl group, which is optionally substituted, or represents a optionally substituted single or condensed heterocyclic group having aromatic properties, having 5 to 12 ring atoms, and containing up to 4 heteroatoms selected from sulfur atoms, oxygen atoms, and nitrogen atoms in a ring or in each ring,
R¹⁰⁰ represents a linear or branched C₁₋₈ alkyl group, a C₃₋₇ cycloalkyl group, a C₄₋₇ cycloalkylalkyl group, an optionally substituted phenyl group or a phenyl C₁₋₆ alkyl group, an optionally substituted 5-membered heteroaromatic ring containing up to 4 heteroatoms selected from oxygen atoms and nitrogen atoms, a hydroxy C₁₋₆ alkyl group, an amino C₁₋₆ alkyl group, a C₁₋₆ alkylaminoalkyl group, a di C₁₋₆ alkylaminoalkyl group, a C₁₋₆ acylaminoalkyl group, a C₁₋₆ alkoxyalkyl group, a C₁₋₆ alkylcarbonyl group, a carboxy group, a C₁₋₆ alkoxycarbonyl group, a C₁₋₆ alkoxycarbonyl C₁₋₆ alkyl group, an aminocarbonyl group, a C₁₋₆ alkylaminocarbonyl group, a di C₁₋₆ alkylaminocarbonyl group, or a halogeno C₁₋₆ alkyl group, or in a case of forming a ring with Ar², R¹⁰⁰ forms a group -(CH₂)ₚ- (where p is 2 or 3),
R¹⁰¹ and R¹⁰² are the same as or different from each other, and each independently represent a hydrogen atom, a C₁₋₆ linear chain or branched alkyl group, or together form a -(CH₂)ₙ₁- group (where n1 is 3, 4, or 5), or R¹⁰¹ and R¹⁰⁰ together form a group -(CH₂)_{q}- (where q is 2, 3, 4, or 5),
R¹¹⁰ is R¹⁰³ or (R⁴⁰⁵)_{q1},
R¹¹¹ is R¹⁰⁴ or a group represented by Formula (2c) or (2g), 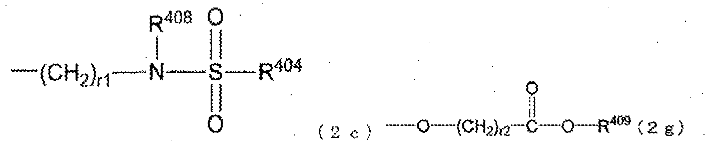
R¹¹² is R¹⁰⁵ or a group represented by Formula (2d), 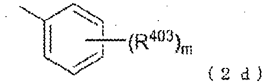
R⁴⁰¹ is selected from a hydrogen atom, a C₁₋₄ alkyl group, a C₃₋₆ cycloalkyl group, and a C₁₋₄ alkylOC(O)-,
A² is a phenyl group or a C₃₋₇ cycloalkyl group,
each R⁴⁰² is independently selected from a hydrogen atom, -OH, -NH₂, -CN, a halogen atom, a C₁₋₆ alkyl group, a C₃₋₇ cycloalkyl group, a C₁₋₆ alkoxy group, and a C₁₋₆ alkoxy C₁₋₆ alkyl group,
n2 is 1, 2, or 3,
each R⁴⁰³ is independently selected from a hydrogen atom, -OH, -NH₂, -NO₂, - CN, a halogen atom, a C₁₋₆ alkyl group, a C₁₋₆ alkoxy group, and a C₁₋₆ alkoxy C₁₋₆ alkyl group,
m is 1, 2, or 3,
r1 is 0, 1, 2, or 3,
r2 is 1, 2, or 3,
R⁴⁰⁴ and R⁴⁰⁹ are selected from a C₁₋₄ alkyl group, a C₁₋₆ alkoxy C₁₋₆ alkyl group, a C₃₋₇ cycloalkyl group, and E-(CH₂)_{b}-, where E is -NR⁴⁰⁶R⁴⁰⁷, -SR⁴⁰⁶, a -SOC₁₋₆ alkyl group, a -SO₂C₁₋₆ alkyl group, N⁺(O⁻)R⁴⁰⁶R⁴⁰⁷, -NR⁴⁰⁶SO₂R⁴⁰⁷, an aryl group, and a N- or C-bonded 5- or 6-membered aromatic or non-aromatic heterocyclic ring having 1, 2, 3, or 4 nitrogen atoms or a N-oxide thereof, and b is 0, 1, 2, 3, 4, or 5,
each R⁴⁰⁵ is independently selected from a hydrogen atom, -OH, -CN, a halogen, -R⁴⁰⁶, -OR⁴⁰⁶, -NR⁴⁰⁶R⁴⁰⁷, -SR⁴⁰⁶, -SOR⁴⁰⁶, and-SO₂R⁴⁰⁶,
ql is 1, 2, or 3,
where R⁴⁰⁶ and R⁴⁰⁷ are each independently selected from a hydrogen atom, a C₁₋₆ linear or branched alkyl group, a C₂₋₆ linear or branched alkenyl group or alkynyl group, and a C₃₋₇ carbocyclic group having 0, 1, or 2 double or triple bonds, where the groups are unsubstituted or substituted with one or more groups selected from -OH, =O, - NH2, -CN, a halogen atom, an aryl, and a C₁₋₃ alkoxy group,
R⁴⁰⁸ is selected from a hydrogen atom, a C₁₋₅ linear or branched alkyl group, and a C₃₋₅ cycloalkyl group, where the groups are unsubstituted or substituted with one or more groups selected from -OH, =O, -NH₂, -CN, a halogen, an aryl group, and a C₁₋₃ alkoxy group,
in a case where R⁴⁰⁴ is E-(CH₂)_{b}-, and E is a N- or C-bonded 5- or 6-membered aromatic or non-aromatic heterocyclic ring or N-oxide thereof, E is unsubstituted or independently selected from -OH, =O, -NH₂, -CN, a halogen atom, a C₁₋₄ alkyl group, a C₁₋₄ alkoxy group, a C₁₋₄ alkyl-CO-, -NR⁴⁰⁶R⁴⁰⁷, an aryl, and a N- or C-bonded 5- or 6-membered aromatic or non-aromatic heterocyclic ring having 1, 2, 3, or 4 nitrogen atoms or a N-oxide thereof,
in a case where R⁴⁰¹, R⁴⁰², R⁴⁰³, or R⁴⁰⁴ is an alkyl group, a cycloalkyl group, an alkoxy group, or an alkoxyalkyl group, the group is unsubstituted or has 1, 2, 3, 4, or 5 substituents each of which is independently selected from -OH, -NH₂, -CN, phenyl, and a halogen,
R¹⁰³ and R¹⁰⁴ are the same as or different from each other, and are independently selected from a hydrogen atom, a C₁₋₆ linear or branched alkyl group, a C₁₋₆ alkenyl group, an aryl group, a C₁₋₆ alkoxy group, a hydroxy group, a halogen atom, a nitro group, a cyano group, a carboxy group, a carboxyamide group, a sulfonamide group, a C₁₋₆ alkoxycarbonyl group, a trifluoromethyl group, an acyloxy group, a phthalimide group, an amino group, a mono- or di-C₁₋₆ alkylamino group, an -O(CH₂)ᵣ-NW₂ group (where r is 2, 3, or 4, and W is a hydrogen atom or a C₁₋₆ alkyl group or forms a group 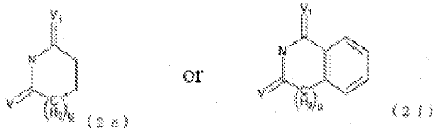
with nitrogen adjacent thereto [in Formula (2e) or (2f), V and V₁ independently represent a hydrogen atom or an oxygen atom, and u is 0, 1, or 2]),
an -O(CH₂)ₜ-OE₂ group (where t is 2, 3, or 4, and E is a hydrogen atom or a C₁₋₆ alkyl group), a hydroxyalkyl group, an aminoalkyl group, a mono- or di-alkylaminoalkyl group, an acylamino group, an alkylsulfonylamino group, an aminoacylamino group, and a mono- or di-alkylaminoacylamino group, up to 4 R¹⁰³ substituents are present in a quinoline nucleus, or R¹⁰⁴ forms a -(CH₂)ₑ- group (where e is 1, 2, or 3) in a case of forming a ring with R¹⁰⁵ as an aryl,
R¹⁰⁵ represents a branched or linear C₁₋₆ alkyl group, a C₃₋₇ cycloalkyl group, a C₄₋₇ cycloalkylalkyl group, an optionally substituted aryl group, or a optionally substituted single or condensed heterocyclic group having aromatic properties, having 5 to 12 ring atoms, and containing up to 4 heteroatoms selected from sulfur atoms, oxygen atoms, and nitrogen atoms in a ring or in each ring, and
X represents an oxygen atom, a sulfur atom, or =N-C≡N], and 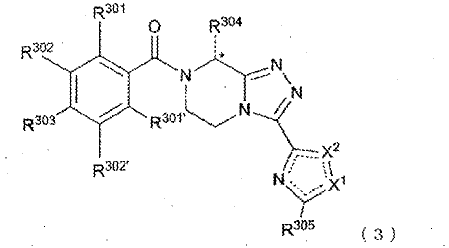
[in Formula (3),
R³⁰¹ is a hydrogen atom, a fluorine atom, or a methyl group,
R^{301'} is a hydrogen atom,
R³⁰² is a hydrogen atom, a fluorine atom, a chlorine atom, or a methoxy group,
R^{302'} is a hydrogen atom or a fluorine atom,
R³⁰³ is a hydrogen atom, a fluorine atom, a chlorine atom, a methyl group, a trifluoromethyl group, or a nitrile group,
R³⁰⁴ is methyl, ethyl, n-propyl, hydroxyethyl, methoxyethyl, trifluoromethyl, difluoromethyl, or fluoromethyl,
R³⁰⁵ is methyl, ethyl, methoxymethyl, trifluoromethyl, difluoromethyl, fluoromethyl, 1-fluoroethyl, 1,1-difluoroethyl, or 2,2,2-trifluoroethyl,
X¹ is a nitrogen atom and X² is a sulfur atom or an oxygen atom, or X¹ is a sulfur atom and X² is a nitrogen atom,
represents a single bond or a double bond, depending on X¹ and X², and *
represents an (R)-enantiomer or racemic compound of the compound of Formula (3)].

6. The prophylactic or therapeutic agent according to Claim 5, wherein the compound represented by Formula (1) is osanetant.

7. The prophylactic or therapeutic agent according to Claim 5, wherein the compound represented by Formula (1) is SSR-146977.

8. The prophylactic or therapeutic agent according to Claim 5, wherein the compound represented by Formula (1) is SSR-241586.

9. The prophylactic or therapeutic agent according to Claim 5, wherein the compound represented by Formula (1) is CS-003.

10. The prophylactic or therapeutic agent according to Claim 5, wherein the compound represented by Formula (2) is talnetant.

11. The prophylactic or therapeutic agent according to Claim 5, wherein the compound represented by Formula (2) is pavinetant.

12. The prophylactic or therapeutic agent according to Claim 5, wherein the compound represented by Formula (2) is SB-235375.

13. The prophylactic or therapeutic agent according to Claim 5, wherein the compound represented by Formula (3) is fezolinetant.

14. The prophylactic or therapeutic agent according to any one of Claims 1 to 3, wherein the neurokinin B signal blocker is an inhibitor of a tachykinin processing enzyme or a decomposition accelerator of a tachykinin processing enzyme.

15. The prophylactic or therapeutic agent according to Claim 14, wherein the inhibitor is an inhibitor of proprotein convertase subtilisin/kexin 1, proprotein convertase subtilisin/kexin 2, carboxypeptidase E, or peptidylglycine alpha-amidating monooxygenase.

16. The prophylactic or therapeutic agent according to any one of Claims 1 to 3, wherein the neurokinin B signal blocker is an expression inhibitor of neurokinin B (NKB), a neurokinin 3 receptor, a tachykinin processing enzyme, a gastrin-releasing peptide (GRP), or a GRP receptor.

17. The prophylactic or therapeutic agent according to any one of Claims 1 to 3, wherein the neurokinin B signal blocker is a removing agent for neurons expressing a neurokinin 3 receptor or a GRP receptor.

18. The prophylactic or therapeutic agent according to Claim 17, wherein the removing agent is GRP, a specific binding substance to a GRP receptor, NKB, or a specific binding substance to a neurokinin 3 receptor, to which a cytotoxic substance is bound.

19. The prophylactic or therapeutic agent according to Claim 18, wherein the cytotoxic substance is a ribosome-inactivating protein or a diphtheria toxin.

20. The prophylactic or therapeutic agent according to Claim 19, wherein the ribosome-inactivating protein is saporin, ricin, or abrin.

21. A prophylactic or therapeutic medicinal composition for an IL-31-mediated disease comprising:
the prophylactic or therapeutic agent according to any one of Claims 1 to 20; and
a pharmaceutically acceptable carrier.

22. A prophylactic or therapeutic medicinal composition for atopic dermatitis comprising:
the prophylactic or therapeutic agent according to any one of Claims 1 to 20; and
a pharmaceutically acceptable carrier.


SEQUENCE LISTING

<110>  Kyushu University
       Toyama University

<120>  Prophylactic or therapeutic agent and pharmaceutical composition
       for IL-31 mediated diseases

<130>  QP180030-PC

<150>  JP2018-215017
<151>  2018-11-15

<150>  JP2019-144913
<151>  2019-08-06

<160>  23

<170>  PatentIn version 3.5

<210>  1
<211>  16
<212>  PRT
<213>  Manduca sexta

<400>  1

Ala Asn Glu Arg Ala Asp Leu Ile Ala Tyr Leu Lys Gln Ala Thr Lys
1               5                   10                  15


<210>  2
<211>  10
<212>  PRT
<213>  Homo sapiens

<400>  2

Asp Met His Asp Phe Phe Val Gly Leu Met
1               5                   10


<210>  3
<211>  25
<212>  DNA
<213>  Artificial Sequence

<220>
<223>  complementary oligonucleotide 1 for CRISPR

<400>  3
caccgagtgc tgagcaaggc tagcg                                           25


<210>  4
<211>  25
<212>  DNA
<213>  Artificial Sequence

<220>
<223>  complementary oligonucleotide 2 for CRISPR

<400>  4

aaaccgctag ccttgctcag cactc 25

<210> 5
<211> 21
<212> DNA
<213> Streptococcus pyogenes

<400> 5
gagtgctgag caaggctagc g 21

<210> 6
<211> 21
<212> DNA
<213> Streptococcus pyogenes

<400> 6
cgctagcctt gctcagcact c 21

<210> 7
<211> 22
<212> DNA
<213> Mus musculus

<400> 7
ctctccccta caaggactct ga 22

<210> 8
<211> 22
<212> DNA
<213> Mus musculus

<400> 8
ccaatctaat cttcagaacg cc 22

<210> 9
<211> 20
<212> DNA
<213> Mus musculus

<400> 9
agcgggcctt cctcactctc 20

<210> 10
<211> 20
<212> DNA
<213> Mus musculus

<400> 10
gggcaccgga ggacaagctg 20

<210> 11
<211> 25
<212> DNA
<213> Mus musculus

<400> 11

ccttgatgcc gttcttctgc ttgtc 25

<210> 12
<211> 24
<212> DNA
<213> Mus musculus

<400> 12
agggagggag ggctcagtaa ggac 24

<210> 13
<211> 25
<212> DNA
<213> Mus musculus

<400> 13
ccaacaggag gaccttacag gcagg 25

<210> 14
<211> 41
<212> DNA
<213> Mus musculus

<220>
<221> misc_feature
<223> Sequence from Fig.6

<400> 14
aaagtgctga gcaaggctag cgtgggtagg acgaggctct g 41

<210> 15
<211> 32
<212> DNA
<213> Mus musculus

<220>
<221> misc_feature
<223> Sequence from Fig.6

<400> 15
gcctgccttc aacaggacca aaggagacat ca 32

<210> 16
<211> 37
<212> DNA
<213> Mus musculus

<220>
<221> misc_feature
<223> Sequence from Fig.6

<400> 16
aaagtgctga gcaaggcgtg ggtaggacga ggctctg 37

<210>  17
<211>  26
<212>  DNA
<213>  Mus musculus

<220>
<221>  misc_feature
<223>  Sequence from Fig.6

<400>  17
aaagtgctga gcaaggctag gctctg                                            26

<210>  18
<211>  8
<212>  PRT
<213>  Mus musculus

<220>
<221>  misc_feature
<223>  Sequence from Fig.6

<400>  18

Lys Val Leu Ser Lys Ala Ser Val
1               5

<210>  19
<211>  6
<212>  PRT
<213>  Mus musculus

<220>
<221>  misc_feature
<223>  Sequence from Fig.6

<400>  19

Gly Pro Lys Glu Thr Ser
1               5

<210>  20
<211>  7
<212>  PRT
<213>  Mus musculus

<220>
<221>  misc_feature
<223>  Sequence from Fig.6

<400>  20

Lys Val Leu Ser Lys Ala Trp
1               5

<210>  21

<211>  6
<212>  PRT
<213>  Mus musculus

<220>
<221>  misc_feature
<223>  Sequence from Fig.6

<400>  21

Asp Gln Arg Arg His His
1               5

<210>  22
<211>  5
<212>  PRT
<213>  Mus musculus

<220>
<221>  misc_feature
<223>  Sequence from Fig.6

<400>  22

Lys Val Leu Ser Lys
1               5

<210>  23
<211>  6
<212>  PRT
<213>  Mus musculus

<220>
<221>  misc_feature
<223>  Sequence from Fig.6

<400>  23

Asp Gln Arg Arg His His
1               5